(19) Europäisches Patentamt / European Patent Office / Office européen des brevets

(11) EP 1 937 619 B1

(12) **FASCICULE DE BREVET EUROPEEN**

(45) Date de publication et mention de la délivrance du brevet: **08.10.2014 Bulletin 2014/41**

(21) Numéro de dépôt: **06807453.3**

(22) Date de dépôt: **20.10.2006**

(51) Int Cl.:
*C07C 59/42* (2006.01) *C07C 59/56* (2006.01)
*A61K 31/20* (2006.01) *A61P 17/00* (2006.01)
*A61Q 19/00* (2006.01)

(86) Numéro de dépôt international: **PCT/EP2006/067641**

(87) Numéro de publication internationale: **WO 2007/045693 (26.04.2007 Gazette 2007/17)**

(54) **NOUVEAUX DERIVES D'HYDROXY-ACIDES GRAS INSATURES ET LEUR UTILISATION DERMO COSMETOLOGIQUE**

NEUE UNGESÄTTIGTE FETTHYDROXYSÄUREDERIVATE UND DEREN DERMOKOSMETOLOGISCHE VERWENDUNG

NOVEL UNSATURATED FATTY HYDROXY ACID DERIVATIVES AND THE DERMOCOSMETOLOGIC USE THEREOF

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **21.10.2005 FR 0510792**

(43) Date de publication de la demande: **02.07.2008 Bulletin 2008/27**

(73) Titulaire: **Pierre Fabre Dermo-Cosmétique
92100 Boulogne-Billancourt (FR)**

(72) Inventeurs:
- **CHARVERON, Marie
F-31000 Toulouse (FR)**
- **TARROUX, Roger
F-31300 Toulouse (FR)**
- **BORDAT, Pascal
F-31320 Mervilla (FR)**

(74) Mandataire: **Regimbeau
20, rue de Chazelles
75847 Paris Cedex 17 (FR)**

(56) Documents cités:
**EP-A- 0 568 307** **EP-A- 0 632 008**
**EP-A1- 0 989 111** **EP-A1- 1 679 071**

- **HANESSIAN, STEPHEN ET AL: "Synthesis of carbocycles from .omega.-substituted .alpha.,.beta.-unsaturated esters via radical -induced cyclizations" CANADIAN JOURNAL OF CHEMISTRY , 65(8), 1859-66 CODEN: CJCHAG; ISSN: 0008-4042, 1987, XP002376764**
- **GUINDON, Y. ET AL: "Stereoselective Hydrogen Transfer Reactions Involving Acyclic Radicals . Tandem Substituted Tetrahydrofuran Formation and Stereoselective Reduction: Synthesis of the C17-C22 Subunit of Ionomycin" JOURNAL OF ORGANIC CHEMISTRY , 59(5), 1166-78 CODEN: JOCEAH; ISSN: 0022-3263, 1994, XP002376741**
- **NISHITANI, KIYOSHI ET AL: "Studies on the terpenoids and related alicyclic compounds. XXXIX. A synthesis of .alpha.-methylene-.gamma.-lactones fused to medium and large rings by intramolecular cyclization of formylated allyl halides" CHEMICAL & PHARMACEUTICAL BULLETIN , 38 (1), 28-35 CODEN: CPBTAL; ISSN: 0009-2363, 1990, XP002376742**
- **BROWN, RICHARD T. ET AL: "Synthesis of methyl secolonitoside" JOURNAL OF THE CHEMICAL SOCIETY, PERKIN TRANSACTIONS 1: ORGANIC AND BIO-ORGANIC CHEMISTRY , (11), 1633-1637 CODEN: JCPRB4; ISSN: 0300-922X, 1997, XP002376743**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

• BARTLETT, PAUL A. ET AL: "A stereoselective total synthesis of the Prelog-Djerassi lactone" JOURNAL OF THE AMERICAN CHEMICAL SOCIETY , 102(1), 337-42 CODEN: JACSAT; ISSN: 0002-7863, 1980, XP002376744

## Description

La présente invention a pour objet de nouveaux dérivés d'hydroxy-acides gras insaturés, ainsi que leur utilisation dermocosmétologique.

De nombreux hydroxy-acides gras saturés sont connus et décrits dans la littérature pour leurs propriétés biologiques et plus particulièrement pour leurs propriétés cosmétiques et pharmacologiques. Par exemple, le principal constituant lipidique de la gelée royale des abeilles est un hydroxy-acide gras insaturé, à savoir l'acide hydroxy-10-décène 2(trans)oïque (Edward E. Smissman et al., 1964. JOU. 29 3517-3520) connu pour son action sur la stimulation du renouvellement épidermique et la lutte contre le vieillissement de la peau, ainsi que pour son utilisation dans des compositions dépigmentantes (EP 1 679 071 et EP 0 989 111).

La demande de brevet EP 0 568 307 décrit des acides alpha-hydroxy-carboxyliques et leur utilisation comme conservateur dans des compositions cosmétiques.

L'article d'Hanessian et al. (Can. J. Chem. 1987, 65, 1859-1866) décrit des hydroxy-acides gras insaturés et leur procédé de préparation mais sans qu'aucune activité biologique n'y soit décrite.

Différents documents de l'état de la technique décrivent des procédés de préparation des hydroxy-acides gras insaturés et de leurs esters (Lee et al., 1993, J. Org. Chem., vol. 58, pages 2918- 2919; Hurd et Saunders, 1952, J. Am. Chem. Soc., vol. 74, pages 5324-5328 ; Krishnamurthy et al., 1989, Indian J. Chem. Sect. A, vol. 28, pages 288-291 ; Plettner et al., 1995, J. Chem, Ecol., vol. 21, pages 1017-1030).

Plus particulièrement, la présente invention concerne des dérivés hydroxy-acides gras insaturés répondant à la formule générale (I) :

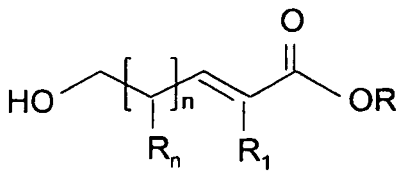

(I)

dans laquelle :

- $R_n$ représente indépendaemment les uns des autres H ou un groupe alkyle linéaire ou ramifié comprenant de 1 à 6 atomes de carbone et étant éventuellement substitué par un atome d'halogène en particulier de fluor,
- $R_1$ représente H, F, Cl, Br, $CF_3$,
- R représente H et
- $3 \leq n \leq 14$.

à la condition toutefois que, lorsque n * 10, l'un au moins des radicaux $R_1$ et $R_n$ ne représente pas l'hydrogène.

Selon une caractéristique particulière de l'invention, les dérivés d'hydroxy-acides gras insaturés de formule générale (I) répondent aux critères suivants :

- $R_1$ représente F, Cl, Br, $CF_3$ ;
- $R_n$ représentent indépendamment les uns des autres H ou un groupe alkyle linéaire ou ramifié comprenant de 1 à 6 atomes de carbone et étant éventuellement substitué par un atome d'halogène en particulier de fluor,
- R représente H et
- $3 \leq n \leq 14$.

Selon une autre caractéristique particulière de l'invention, les dérivés d'hydroxy-acides gras insaturés de formule générale (I) répondent aux critères suivants :

- $R_n$ représente indépendamment les uns des autres H ou un groupe alkyle linéaire ou ramifié comprenant de 1 à 6 atomes de carbone et étant éventuellement substitué par un atome d'halogène en particulier de fluor,
- $R_1$ représente H, F, Cl, Br, $CF_3$,
- R représente H et
- $6 \leq n \leq 14$,

à la condition toutefois que, lorsque n ≠ 10, l'un au moins des radicaux $R_1$ et $R_n$ ne représente pas l'hydrogène.

Selon une autre caractéristique particulière de l'invention, les dérivés d'hydroxy-acides gras insaturés de formule générale (I) répondent aux critères suivants :

- $R_1$ = F, Cl, Br ou $CF_3$,
- R=$R_n$=H, et
- 3≤n≤14.

Selon une autre caractéristique particulière de l'invention, les dérivés d'hydroxy-acides gras insaturés de formule générale (I) répondent aux critères suivants :

- $R_1$ représente F, Cl, Brou $CF_3$,
- au moins l'un des radicaux $R_n$ représentant un groupe alkyle linéaire ou ramifié comprenant de 1 à 6 atomes de carbone et étant éventuellement substitué par un atome d'halogène en particulier de fluor,
- R représente H, et
- 3≤n≤14,

Selon une autre caractéristique particulière de l'invention, les dérivés d'hydroxy-acides gras insaturés de formule générale (I) répondent aux critères suivants :

- $R_1$=F,
- R=$R_n$=H, et
- 3≤n≤14.

Selon une autre caractéristique particulière de l'invention, les dérivés d'hydroxy-acides gras insaturés de formule générale (I) répondent aux critères suivants :

- $R_1$=F,
- R=$R_n$=H, et
- n=13.

Selon une autre caractéristique particulière de l'invention, les dérivés d'hydroxy-acides gras insaturés de formule générale (I), répondent aux critères suivants :

- $R_1$=F,
- R=$R_n$=H, et
- n=6.

Selon une autre caractéristique particulière de l'invention, les dérivés d'hydroxy-acides gras insaturés de formule générale (I) répondent aux critères suivants :

- R=$R_1$=H
- un seul des radicaux $R_n$ représente un groupe méthyle, les autres étant des atomes d'hydrogène, et,
- n=4.

Selon une autre caractéristique particulière de l'invention, les dérivés d'hydroxy-acides gras insaturés de formule générale (I) répondent aux critères suivants :

- $R=R_1=R_n=H$ et
- n=10.

Selon une autre caractéristique particulière de l'invention, les dérivés d'hydroxy-acides gras insaturés de formule générale (I) répondent aux critères suivants :

- $R_1=F$,
- $R=R_n=H$, et
- n=10.

Selon une autre caractéristique particulière de l'invention, les dérivés d'hydroxy-acides gras insaturés de formule générale (I) répondent aux critères suivants :

- $R_1=F$,
- $R=R_n=H$, et
- n=8.

Selon la présente invention les dérivés d'hydroxy-acides gras insaturés de formule générale (I) précédemment définis peuvent être utilisés à titre de principe actif en association avec un excipient approprié pour réaliser des compositions dermocosmétologiques destinée à traiter des troubles de la pigmentation.

Selon la présente invention, les dérivés d'hydroxy-acides gras insaturés de formule générale (I) sont plus particulièrement destinés à des compositions destinées à provoquer un éclaircissement de la peau.

Selon la présente invention les dérivés d'hydroxy-acides gras insaturés de formule générale (I) suivante :

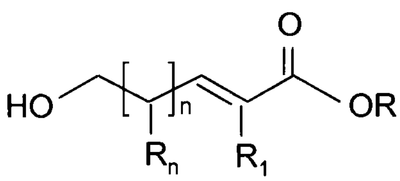

(I)

dans laquelle :

- $R_n$ représente indépendamment les uns des autres H ou un groupe alkyle linéaire ou ramifié comprenant de 1 à 6 atomes de carbone et étant éventuellement substitué par un atome d'halogène en particulier de fluor,
- $R_1$ représente H, F, Cl, Br, $CF_3$,
- R représente H ou un groupe alkyle linéaire ou ramifié comprenant de 1 à 6 atomes de carbone et étant éventuellement substitué par un atome d'halogène en particulier de fluor, et
- $3 \leq n \leq 14$.

Selon une caractéristique particulière de l'invention, les dérivés d'hydroxy-acides gras insaturés de formule générale (I) répondent aux critères suivants :

- R représente H,
- $R_n$ représente indépendamment les uns des autres H ou un groupe alkyle linéaire ou ramifié comprenant de 1 à 6 atomes de carbone et étant éventuellement substitué par un atome d'halogène en particulier de fluor,

- $R_1$ représente H, F, Cl, Br, $CF_3$, et
- $3 \leq n \leq 14$,

peuvent être utilisés à titre de principe actif en association avec un excipient approprié pour réaliser des compositions dermocosmétologiques à activité anti-radicalaire, anti-inflammatoire, anti-purigineuse et/ou destinée à traiter des troubles de la kératinisation et de la pigmentation et/ou à améliorer la cicatrisation.

Les dérivés d'hydroxy-acides gras insaturés de formule générale (I) suivante :

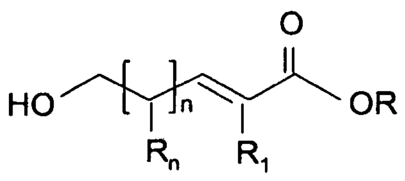

(I)

dans laquelle :

- $R_n$ représente indépendamment les uns des autres H ou un groupe alkyle linéaire ou ramifié comprenant de 1 à 6 atomes de carbone et étant éventuellement substitué par un atome d'halogène en particulier de fluor,
- $R_1$ représente H, F, Cl, Br, $CF_3$,
- R représente H ou un groupe alkyle linéaire ou ramifié comprenant de 1 à 6 atomes de carbone et étant éventuellement substitué par un atome d'halogène en particulier de fluor, et
- $3 \leq n \leq 14$.

Selon une caractéristique particulière de l'invention, les dérivés d'hydroxy-acides gras insaturés de formule générale (I) répondent aux critères suivants :

- R représente H,
- $R_n$ représente indépendamment les uns des autres H ou un groupe alkyle linéaire ou ramifié comprenant de 1 à 6 atomes de carbone et étant éventuellement substitué par un atome d'halogène en particulier de fluor,
- $R_1$ représente H, F, Cl, Br, $CF_3$, et
- $3 \leq n \leq 14$,

sont plus particulièrement destinés à des compositions destinées au traitement du psoriasis, du prurit et/ou de la dermite atopique, ainsi qu'au traitement du vieillissement cutané et des taches de vieillesse blanches ou brunes.

- En raison de leur activité anti-radicalaire, les dérivés d'hydroxy-acides gras insaturés de formule générale (I) sont également utiles pour éviter ou limiter la photocarcinogénèse cutanée à des stades précoces et donc peuvent être utilisés dans la prévention et le traitement de diverses maladies tumorales de la peau.

Les dérivés d'hydroxy-acides gras insaturés de formule générale (I) peuvent être préparés :

- par la mise en oeuvre de la réaction de Wittig Horner par la réaction d'un phosphonate de formule (III) suivante :

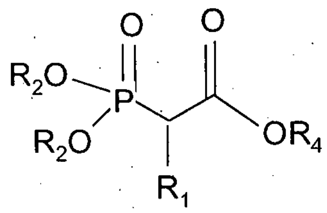

(III)

dans laquelle :

- $R_1$ est tel que défini à propos de la formule (I) ;
- $R_2$ représente un groupe alkyle, linéaire ou ramifié, comprenant de 1 à 6 atomes de carbone, et est de préférence un groupe éthyle ou méthyle, lesdits groupes $R_2$ pouvant former un cycle avec les atomes d'oxygène des groupes $OR_2$ et l'atome de phosphore adjacent, et
- $R_4$ représente un groupe alkyle, linéaire ou ramifié, comprenant de 1 à 6 atomes de carbone, et est de préférence un groupe éthyle.

- ou, lorsque $R_1$ = H, par la mise en oeuvre de la réaction de Doebner-Knoevenagel par la réaction d'un dérivé d'acide malonique de formule $R_4OOC-CH_2COOR$, R étant tel que défini ci-dessus à propos de la formule (I), sur un lactol de formule (II) suivante :

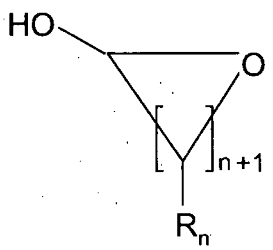

(II)

$R_n$ et n étant tels que définis ci-dessus,
afin d'obtenir un hydroxy-ester de formule (IV) suivante :

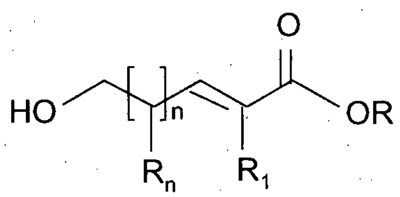

(IV)

dans laquelle n, R et $R_1$ sont tels que définis ci-dessus,
et éventuellement, lorsque R représente un groupe $R_4$ tel que défini ci-dessus, une réaction de saponification de l'hydroxy-ester de formule (IV) susmentionnée, pour obtenir un dérivé d'hydroxy-acide de formule (I) correspondante.
La présente invention sera illustrée dans le cadre des exemples de synthèse donnés ci-après :

Exemple 1 Mode opératoire pour la synthèse du DHA

1. Etape 1 : préparation de l'Oxonan-2-one

43,5g (345 mmol) de cyclooctanone sont mis en solution dans 430ml de dichloroéthane. 170g (985 mmol) d'acide *meta*-chloroperbenzoïque sont ensuite ajoutés: Le milieu est chauffé à 80°C pendant 48 heures. A température ambiante, 400ml d'une solution saturée $Na_2S_2O_5$ et $NaHCO_3$ (1/1 v/v) sont ajoutés. Le milieu est agité vigoureusement pendant 18h. La phase organique est séparée et mise en présence de KI et $H_2O$ pendant 6h. La phase organique est séparée et lavée par une solution saturée de $Na_2S_2O_3$, par une solution saturée de NaCl, puis est séchée sur $MgSO_4$, filtrée et concentrée sous vide pour donner un produit brut de 36g.

La lactone est purifiée par empâtage dans le pentane (60ml), puis par filtration du précipité acide *meta*-chlorobenzoïque formé à froid, m=26,6g (54%).

La lactone obtenue est un composé de formule :

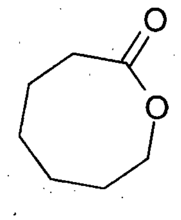

Caractérisation :

CCM : Rf = 0,3 (heptane / acétate d'éthyle 7/3)

$^1$H RMN (300MHz, $CDCl_3$) : δ 1.42-1.49 (m, 4H), 1.65-1.75 (m, 6H), 2.30 (t, *J*=5.4Hz, 2H), 4.30 (t, *J*=5.7Hz, 2H).

2. Etape de réduction partielle en lactol

26,6g (187,2 mmol) de lactone sont dilués dans 210ml de toluène, sous atmosphère d'azote. Le milieu est refroidi à -78°C et 156,4ml (189.1 mmol) de Dibal-H en solution à 20% dans le toluène sont additionnés goutte à goutte, en maintenant la température à -78°C. Le mélange est agité pendant 2h à -78°C. 200ml d'une solution saturée de sels de Rozen sont ajoutés à -78°C. Après 18h d'agitation vigoureuse à température ambiante, le mélange biphasique est filtré sur célite, puis extrait à l'acétate d'éthyle. Les phases organiques sont lavées avec une solution saturée de NaCl, séchées sur $MgSO_4$, filtrées et concentrées sous vide pour donner un brut de 26g (dont 25% de dérivé diol, soit un rendement estimé à 72%). Le lactol, en équilibre forme ouverte, forme cyclique, est utilisé ainsi, sans purification supplémentaire.

Caractérisation :

CCM : Rf = 0,4 (heptane / acétate d'éthyle 6/4)

$^1$H RMN (300MHz, $CDCl_3$) : δ 1.34-1.68 (m, 10H), 2.45 (t, *J*=5.4Hz, 2H), 3.66 (t, *J*=6.6Hz, 2H), 9.78 (t, J=1.8Hz, 1H).

3. Etape 3 : Réaction de Wittig-Horner

19g (131.8 mmol) de lactol sont dilués dans 250 ml d'éthanol. 31,4ml (158,1 mmol) de triéthylphosphonacétate sont additionnés au milieu en présence de 27,3g (197,5 mmol) de carbonate de potassium. Le milieu réactionnel est chauffé à 40°C pendant 18 heures. A température ambiante, le milieu est hydrolysé par 200ml d'eau distillée et extrait avec de l'acétate d'éthyle. Les phases organiques sont lavées par une solution saturée de NaCl, séchées sur $MgSO_4$, filtrées et concentrées sous vide pour donner un produit brut de 20g.

L'ester obtenu est purifié par chromatographie avec une élution heptane / acétate d'éthyle 7/3 : 15g de produit sont obtenus (53% de rendement).

L'ester obtenu est un composé de formule :

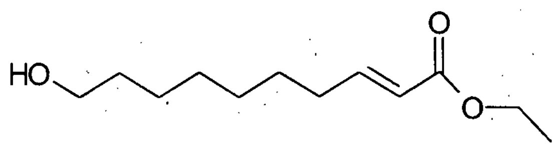

Caractérisation :

CCM : Rf = 0,4 (heptane / acétate d'éthyle 7/3)
$^1$H RMN (300MHz, $CDCl_3$) : δ 1.24-1.38 (m, 9H), 1.43-1.50 (m, 2H), 1.51-1.57 (m, 2H), 2.15-2.21 (q, 2H), 3.60-3.64 (t, 2H), 4.14-4.20 (t, 2H), 5.77-5.82 (d, *J*=15.6Hz, 1 H), 6.91-6.98 (dt, J=15.6Hz, 1 H).

4. Etape 4 : Réaction de saponification

0.60g (2.81 mmol) d'hydroxy-ester sont solubilisés dans 10 volumes de tétrahydrofurane. Lentement, 3.4ml (6.75 mmol) d'une solution de soude 2M sont additionnés. Le milieu est chauffé à 65°C pendant 3h. Une fois la réaction terminée, le milieu est hydrolysé par l'ajout d'une solution d'acide chlorhydrique 3M, jusqu'à l'obtention d'un pH = 2. Le mélange est concentré à sec et la phase aqueuse est alors extraite par de l'acétate d'éthyle. Les phases organiques sont lavées par une solution saturée de NaCl, séchées sur $MgSO_4$, filtrées et concentrées sous vide pour donner un produit brut de 0.6g.

Le dérivé d'hydroxy-acide insaturé attendu est obtenu, sous forme d'un solide blanc, par recristallisation dans l'acétonitrile à froid, m = 0.37g (71%).

Le dérivé d'hydroxy-acide obtenu est un composé de formule :

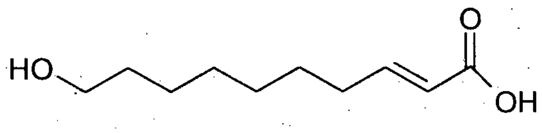

Caractérisation :

CCM : Rf = 0,1 (heptane / acétate d'éthyle 6/4)
$^1$H RMN (300MHz, $CDCl_3$) : δ 1.33-1.37 (m, 6H), 1.45-1.49 (m, 2H), 1.55-1.58 (m, 2H), 2.20-2.25 (q, 2H), 3.62-3.66 (t, 2H), 5.79-5.84 (d, *J*=15.6Hz, 1H), 7.03-7.10 (dt, *J*=15.6Hz, 1H)
Spectroscopie de Masse : $[M-Na]^+$ 209 (calculé 186)
Point de fusion : 62.5 °C$^+_-$1°C

<u>Exemple 2 Mode opératoire pour la synthèse du DHA α-fluoré l'acide 10-Hydroxy-dec-2-fluoro-2-enoïgue : (R1=F, n=6)</u>

Seule l'étape 3 est modifiée : à partir du lactol (0.89g, 7.7mmol), la réaction de Wittig-Horner est conduite en présence de Diéthylphosphonofluoroacétate de méthyle (2.1 g, 9.2mmol) et de carbonate de potassium (1.6g, 11.5mmol) dans l'éthanol (9ml) à 40°C. L'étape 4 est conduite selon le protocole précédent. Le produit obtenu après recristallisation, est sous forme d'un mélange *cis/trans.*

Le dérivé d'hydroxy-acide obtenu est un composé de formule :

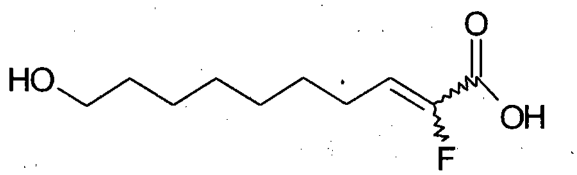

Caractérisation :

CCM : Rf = 0,1 (heptane / acétate d'éthyle 6/4)
$^1$H RMN (300MHz, $CDCl_3$) : δ 1.36-1.6 (m, 20H), 2.27-2.30 (m, 2H, forme *cis),* 2.50-2.58 (m, 2H, forme *trans),* 3.69 (m, 4H), 6.05 (dt, J=21.6Hz, 1H, forme *trans)* et 6.25 (dt, *J*=40.8Hz, 1 H, forme *cis).*
Spectroscopie de Masse : $[M-Na]^+$ 227 (calculé 204)

<u>Exemple 3 Mode opératoire pour la synthèse de l'acide 9-hydroxy-nona-2*t*-enoïque</u>

Le protocole de synthèse du DHA est appliqué à la cycloheptanone pour conduire à l'acide 9-hydroxy-nona-2*t*-enoïque.

Caractérisation :

CCM : Rf = 0,1 (heptane / acétate d'éthyle 6/4)
$^1$H RMN (300MHz, $CDCl_3$) : δ 1.36-1.61 (m, 8H), 2.22-2.27 (m, 2H), 3.67 (t, *J*=6.3Hz, 2H), 5.84 (dt, *J*=15.6Hz, 1H), 7.09 (dt, *J*=15.6Hz, 1H).
Spectroscopie de Masse : $[M\text{-}Na]^+$ 195 (calculé 172)

Exemple 4 Mode opératoire pour la synthèse de l'acide 11-hydroxy-undeca-2*t*-enoïque

Le protocole de synthèse du DHA est appliqué à la cyclononanone pour conduire à l'acide 11-hydroxy-undeca-2*t*-enoïque.

Caractérisation :

CCM : Rf = 0,1 (heptane / acétate d'éthyle 6/4)
$^1$H RMN (300MHz, $CDCl_3$) : δ 1.33-1.61 (m, 12H), 2.20-2.28 (m, 2H), 3.66 (t, *J*=6.0Hz, 2H), 5.84 (dt, *J*=15.9Hz, 1 H), 7.09 (dt, *J*=15.9Hz, 1 H).
Spectroscopie de Masse : $[M\text{-}Na]^+$ 223 (calculé 200)

Exemple 5 Mode opératoire pour la synthèse de l'acide 12-hydroxy-dodeca-2*t*-enoïque

Le protocole de synthèse du DHA est appliqué à la cyclodecanone pour conduire à l'acide 12-hydroxy-dodeca-2*t*-enoïque.
Caractérisation :

CCM : Rf = 0,1 (heptane / acétate d'éthyle 6/4)
$^1$H RMN (300MHz, $CDCl_3$) : δ 1.35-1.56 (m, 14H), 2.20-2.27 (m, 2H), 3.55 (t, *J*=6.6Hz, 2H), 5.80 (dt, *J*=15.6Hz, 1 H), 6.96 (dt, *J*=15.6Hz, 1 H). Spectroscopie de Masse : $[M\text{-}Na]^+$ 237 (calculé 214)

Exemple 6 Mode opératoire pour la synthèse de l'acide 12-hydroxy-dodeca-2-fluoro-2*t*-enoïque

Le protocole de synthèse du DHA fluoré en position 2 (exemple 2) est appliqué à la cyclodecanone pour conduire à l'acide 12-hydroxy-dodec-2-fluoro-2-enoïque sous forme d'un mélange *cis*/*trans.*

Caractérisation :

CCM : Rf = 0,1 (heptane / acétate d'éthyle 6/4)
$^1$H RMN (300MHz, $CDCl_3$) : δ 1.34-1.56 (m, 14H), 2.24-2.27 (m, 2H forme *cis)* ou 2.49-2.54 (m, 2H structure *trans),* 3.55 (t, *J*=6.6Hz, 2H), 5.97(dt, *J*=21.9Hz, 1H structure *trans)* ou 6.10 (dt, *J*=55.2Hz, 1H, forme *cis).*
Spectroscopie de Masse : $[M\text{-}Na]^+$ 255 (calculé 232)

Exemple 7 Mode opératoire pour la synthèse de l'acide 13-hydroxy-tridec-2*t*-enoïque

Le protocole de synthèse du DHA est appliqué à l'oxacyclododecan-2-one pour conduire à l'acide 13-hydroxy-tridec-2*t*-enoïque.
Caractérisation :

CCM : Rf = 0,1 (heptane / acétate d'éthyle 6/4)
$^1$H RMN (300MHz, $CDCl_3$) : δ 1.27-1.63 (m, 16H), 2.21-2.28 (m, 2H), 3.64 (t, *J*=3.6Hz, 2H), 5.84 (dt, *J*=15.6Hz, 1H), 7.09 (dt, *J*=15.6Hz, 1H).
Spectroscopie de Masse : $[M\text{-}Na]^+$ 251 (calculé 228)

Exemple 8 Mode opératoire pour la synthèse de l'acide 14-hydroxy-tetradec-2*t*-enoïque

Le protocole de synthèse du DHA est appliqué à l'oxacyclotridecan-2-one pour conduire à l'acide 14-hydroxy-tetradec-2*t*-enoïque.

Caractérisation :

CCM : Rf = 0,1 (heptane / acétate d'éthyle 6/4)
$^1H$ RMN (300MHz, $CDCl_3$): $\delta$ 1.29-1.35 (m, 14H), 1.46-1.61 (m, 4H), 2.21-2.29 (m, 2H), 3.66 (t, *J*=6.6Hz, 2H), 5.84 (dt, *J*=15.6Hz, 1H), 7.09 (dt, *J*=15.6Hz, 1 H).
Spectroscopie de Masse : $[M-Na]^+$ 265 (calculé 242)

Exemple 9 Mode opératoire pour la synthèse de l'acide 14-hydroxy-tetradec-2-fluoro- 2-enoïque

Le protocole de synthèse du DHA fluoré en position 2 (exemple 2) est appliqué à l'oxacyclotridecan-2-one pour conduire à l'acide 14-hydroxy-tetradec-2-fluoro-2-enoïque sous forme d'un mélange *cis*/*trans.*

Caractérisation :

CCM : Rf = 0,1 (heptane / acétate d'éthyle 6/4)
$^1H$ RMN (300MHz, $CDCl_3$) : $\delta$ 1.29 (m, 28H), 1.42-1.62 (m, 8H), 2.27-2.31 (m, 2H, forme *cis),* 2.51-2.56 (m, 2H, forme *trans),* 3.68 (t, *J*=6.6Hz, 2H), 3.70 (t, *J*=6.6Hz, 2H), 6.04 (dt, *J*=21.3Hz, 1H, forme *trans),* 6.27 (dt, *J*=33.0Hz, 1H, forme *cis).*
Spectroscopie de Masse : $[M-Na]^+$ 283 (calculé 260)

Exemple 10 Mode opératoire pour la synthèse de l'acide 17-hydroxy-heptadec-2*t*-enoïque

Le protocole de synthèse du DHA est appliqué au cyclopentadecanolide pour conduire à l'acide 17-hydroxy-heptadec-2*t*-enoïque.

Caractérisation :

CCM : Rf = 0,1 (heptane / acétate d'éthyle 6/4)
$^1H$ RMN (300MHz, $CDCl_3$) : $\delta$ 1.27-1.32 (m, 20H), 1.46-1.61 (m, 4H), 2.20-2.29 (m, 2H), 3.67 (t, *J*=6.6Hz, 2H), 5.84 (dt, *J*=15.6Hz, 1 H), 7.08 (dt, *J*=15.6Hz, 1 H).
Spectroscopie de Masse : $[M-Na]^+$ 307 (calculé 284)

Exemple 11 Mode opératoire pour la synthèse de l'acide 17-hydroxy-heptadec-2-fluoro- 2-enoïque

Le protocole de synthèse du DHA fluoré en position 2 (exemple 2) est appliqué au cyclopentadecanolide pour conduire à l'acide 17-hydroxy-heptadec-2-fluoro-2-enoïque sous forme d'un mélange, *cis*/*trans.*

Caractérisation :

CCM : Rf = 0,1 (heptane / acétate d'éthyle 6/4)
$^1H$ RMN (300MHz, $CDCl_3$) : $\delta$ 1.27 (m, 40H), 1.45-1.62 (m, 8H), 2.24-2.32 (m, 2H, forme *cis),* 2.50-2.58 (m, 2H, forme *trans),* 3.69 (t, *J*=6.6Hz, 4H), 6.05 (dt, J=21.6Hz, 1 H, forme *trans)* et 6.26 (dt, J=40.8Hz, 1 H, forme *cis*).
Spectroscopie de Masse : $[M-H]^-$ 301 (calculé 302)
Point de fusion : 77°C $\pm$ 1°C

Exemple 12 Mode opératoire pour la synthèse de l'acide 18-hydroxy-octadec-2*t*-enoïque : (R1=H, n=14)

Le protocole de synthèse du DHA est appliqué à l'hexacyclodecanolide pour conduire à l'acide 18-hydroxy-octadec-2*t*-enoïque.

Caractérisation :

CCM : Rf = 0,1 (heptane / acétate d'éthyle 6/4)
$^1$H RMN (300MHz, $CDCl_3$) : $\delta$ 1.27-1.65 (m, 26H), 2.19-2.39 (m, 2H), 3.67 (t, *J*=6.6Hz, 2H), 5.83 (dt, *J*=15.6Hz, 1H), 7.09 (dt, *J*=15.6Hz, 1H).
Spectroscopie de Masse : $[M-Na]^+$ 321 (calculé 298)

Exemple 13 : caractérisation de l'acide 16-hydroxy-hexadec-2*t*-enoïque

Caractérisation :

CCM : Rf = 0,1 (heptane / acétate d'éthyle 6/4)
$^1$H RMN (300MHz, $CDCl_3$) : $\delta$ 1.27-1.32 (m, 18H), 1.46-1.61 (m, 4H), 2.21-2.28 (m, 2H), 3.66 (t, *J*=6.6Hz, 2H), 5.85 (dt, *J*=15.6Hz, 1H), 7.09 (dt, *J*=15.6Hz, 1H).
Spectroscopie de Masse : $[M-Na]^+$ 293 (calculé 270).

Exemple de réaction de Doebner-Knovenagel à partir de l'oxacyclotridecanol :

1.4g (7.0 mmol) d'oxacyclotridecanol (obtenu selon le protocole de réduction partielle en lactol) sont mis en solution, sous flux d'azote, dans 4 volumes de pyridine, en présence de 1.09g (10.5 mmol) d'acide malonique et 0.11 ml de piperidine. Le milieu réactionnel est chauffé à 80°C pendant 1h30, puis au reflux pendant 2h. A température ambiante, la solution est versée sur une solution HCl 3M (50 ml). Le solide filtré est lavé par de l'eau puis est recristallisé dans l'acétonitrile (1.2g obtenu soit 70% de rendement).
Les dérivés d'hydroxy-acides gras insaturés selon l'invention ont fait l'objet d'un certain nombre d'expémimentations qui ont démontré leur intérêt comme principe actif dans des compositions dermatologiques et/ou cosmétiques.

Etude de l'effet anti-radicalaire sur espèces actives de l'oxygène (EAO)

Durant le métabolisme cellulaire normal, lors d'exposition occasionnelle de la peau à des agents stressants ou au cours de pathologies dermatologiques, des espèces oxygénées réactives encore appelées Espèces Activées de l'Oxygène (EAO) sont générées (Y. M. W. Janssen *et al,* 1993). Ces EAO, décrites comme des métabolites très réactifs, jouent un rôle important dans bon nombre de processus tels que l'inflammation, le vieillissement et la promotion tumorale.
Les EAO sont considérées comme les « seconds messagers » dans la signalisation cellulaire du stress oxydatif et donc comme les médiateurs précoces de l'inflammation (A. Van Der Vliet and A. Bast, 1992).
Leur surproduction induit d'importants dommages au sein de la cellule. Certains constituants cellulaires sont alors les cibles majeures d'un tel stress oxydatif : les composants lipidiques de la membrane plasmique (lipoperoxydation), les protéines (dénaturation et dégradation) et le matériel génétique ou ADN (mutations) peuvent être altérés. Les cellules sont capables de limiter ces dommages oxydatifs grâce à différents systèmes de défense antiradicalaire (antioxydants enzymatiques et non enzymatiques) (B. P. Yu, 1994 ; H. Steiling *et al,* 1999).
Cependant, dans certaines conditions, les EAO sont produites en quantité telle que l'activité antioxydante cellulaire est insuffisante ; ces EAO deviennent alors des facteurs inducteurs de pathologies inflammatoires et du vieillissement tissulaire (Y. Miyachi *et al,* 1986 ; M. Kress *et al,* 1995).
Il existe différents agents chimiques (ex : $H_2O_2$) ou physiques (ex : UVA) capables de générer *in vitro* un stress oxydatif. Ainsi les EAO produites vont altérer diverses cibles cellulaires (Membranes, ADN ou Protéines) dont l'altération peut-être analysée par des méthodologies biochimiques largement utilisées comme le dosage des TBARS pour la lipoperoxydation lipidique, ou le dosage *in vitro* des EAO intracellulaires à l'aide de la sonde $H_2$DCF-DA.
Nous avons mis en place un modèle d'étude *in vitro* du stress oxydatif induit par $H_2O_2$ / Fer, $H_2O_2$ qui génère massivement des EAO intracellulaires par une réaction en chaîne déclenchée par l'oxydation des lipides membranaires.
Cette technique est basée sur l'utilisation d'une sonde fluorescente, la 6-carboxy-2',7'-dichlorodihydro fluorescein diacetate, di (acetoxymethyl ester) ($H_2$DCF-DA), qui, une fois qu'elle a pénétré dans la cellule, est désacétylée par les estérases intracellulaires formant alors du $H_2$DCF. Ce produit est oxydé par les EAO intracellulaires en un composé hautement fluorescent: la 2',7'-dichlorofluorescein (DCF) *(*Suematsu M et al., 1996, Free Radicals Pratical Approach, Punchard ed. p83-99*).*

Les matériel et méthodes utilisés pour le dosage *in vitro* des EAO intracellulaires sont indiqués ci-après.

a) Outil cellulaire:

Lignée cellulaire de fibroblastes murins cutanés L929.

b) Matériel:

- Microplaque 96 puits à fond plat
- Cytofluorimètre Cytofluor II : Réf. PERSEPTIVE BIOSYSTEMS c) Réactifs :

Réactifs de culture cellulaire :

- Milieu de culture Dulbecco's Modified Eagle Médium (DMEM)
- Sérum de veau foetal (SVF)
- Tampon phosphate PBS pH 7,4
- Trypsine EDTA (1X)

Sonde fluorescente :

- 6-carboxy-2',7'-dichlorodihydrofluorescein diacetate, di(acetoxymethyl ester) (PM. 675,43)

Produits de stimulation des cellules :

- Peroxyde d'Hydrogène ($H_2O_2$) 3% : Réf. GIFRER (Laboratoire Gifrer Barbezat)
- Ferrous Ammonium Sulfate ($Fe^{2+}$)
- Ferric Ammonium Sulfate ($Fe^{3+}$)

d) Produits testés :

Les concentrations testées sont des concentrations non cytotoxiques. La cytotoxicité a été réalisée par la méthode du rouge neutre, après incubation du produit durant 3 heures.

Le produit anti-radicalaire de référence est la vitamine E ou $\alpha$ tocophérol (PM : 430.7) (SIGMA, réf : T-1539)

La solution mère est préparée à 400mg/ml dans du DMSO et conservée à -20°C. La solution de pré-traitement est préparée extemporanément à 400$\mu$g/ml dans du milieu de culture sans SVF.

Pour l'évaluation des dérivés d'hydroxy-acides gras insaturés, les dilutions sont préparées dans du milieu de culture extemporanément, pour une gamme de concentrations de 0.02, 0.2, 2 et 20 ng/ml.

e) Protocole :

Ensemencement des cellules :

Les cellules de la lignée de fibroblastes L929 sont ensemencées dans des microplaques de 96 puits à fond plat dans 100$\mu$l de DMEM supplémenté de 10% de SVF, puis elles sont incubées une nuit à 37°C en atmosphère humide à 5% de $CO_2$.

Le blanc de plaque sans cellules est évalué sur 6 puits. Pré-traitement des cellules :

Les dilutions des produits à tester et molécule de référence sont réalisées dans le milieux de culture sans SVF, puis déposées dans 7 puits à raison de 100µl par puits.

Les cellules sont alors incubées pendant 3 heures à 37°C en atmosphère humide à 5% de $CO_2$.

Les cellules « Témoin » (fluorescence naturelle des cellules), « Contrôle » (production basale de EAO) et « Stimulées » (production de EAO après traitement oxydatif) sont recouvertes avec 100µl de DMEM.

Les cellules « Contrôle » sont incubées avec la sonde mais ne sont ni prétraitées ni traitées.

Les cellules « Stimulées » sont incubées avec la sonde et sont traitées mais pas prétraitées.

Les cellules « Témoin » ne sont ni pré-traitées, ni incubées avec la sonde, ni traitées.

Incubation des cellules avec la sonde et stress oxydatif :

Les cellules sont rincées au PBS 1X à raison de 100µl par puits. Puis elles sont mises à incuber 30 minutes à 37°C en atmosphère humide à 5% de $CO_2$ avec 50µl de sonde $H_2$DCF-DA à 5µM.

Après 30 minutes au contact de la sonde seule, les cellules sont mises à incuber 30 minutes à 37°C en atmosphère humide à 5% de $CO_2$ avec un ajout de 25µl d'$H_2O_2$ à 800µM et 25µl de solution de fer ferreux et ferrique à 8mM, pour avoir des concentrations finales de 200µM d'$H_2O_2$ et 2mM de fer ferreux et ferrique.

Les cellules sont ensuite rincées au PBS 1X à raison de 100µl par puits, puis incubées 30 minutes à 37°C en atmosphère humide à 5% de $CO_2$ avec 100µl de PBS 1X.

Ces 1 h 30 minutes d'incubation à 37°C permettent aux estérases intracellulaires de désacétyler la sonde en $H_2$DCF, oxydable par les EAO intracellulaire en DCF : composé fluorescent dont la formation est proportionnelle à la quantité d'EAO intracellulaires.

L'intensité de fluorescence est lue au cytofluorimètre à λexcitation = 485nm et λémission = 530nm. Elle reflète la quantité d'EAO intracellulaires produite.

Schéma du protocole d'étude :

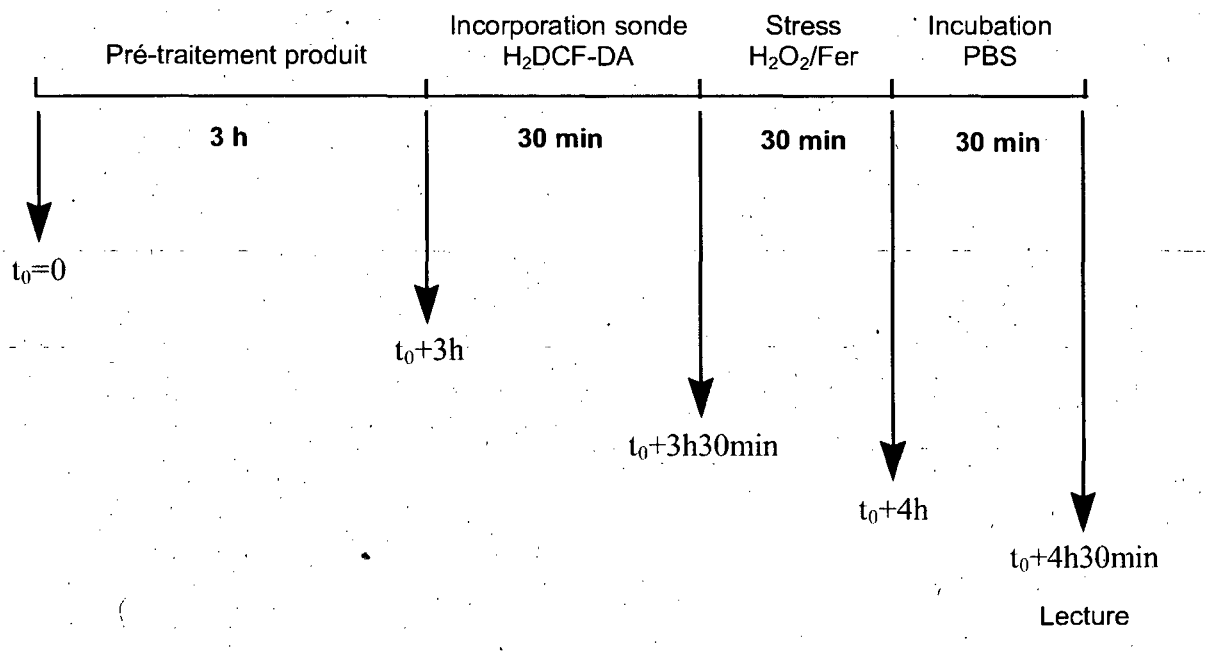


Calcul du % de protection contre la production d'EAO intracellulaires

Le rapport ci-dessous permet de calculer pour chaque concentration de produit testé le % de protection contre la production d'EAO intracellulaires (puisque l'Intensité de Fluorescence ou IF exprime la libération intracellulaire d'EAO).

$$\frac{[\text{IF Oxydant } (H_2O_2/\text{Fer}) - \text{IF (Oxydant + Produit)}] \times 100}{\text{IF Oxydant } (H_2O_2/\text{Fer}) - \text{IF Contrôle}}$$

Les valeurs indiquées dans le tableau ci-après sont les pourcentages d'inhibition de production d'EAO intracellulaire suite à un stress oxydatif exogène, par rapport aux cellules « témoin » (100%) et aux cellules « stimulé » (0%).

Les pourcentages moyens de protection, pour les 13 molécules testées à 4 concentrations sur la lignée L929 sont présentés dans le tableau I ci-dessous.

Tableau I : Analyse de la Protection Contre la Production Intracellulaire d'EAO pour les 13 molécules dérivés d'hydroxy acides gras insaturés : Pourcentage d'inhibition de la production intracellulaire d'EAO

| Vit. E 400µg/ml | | | 47 % | | | | |
|---|---|---|---|---|---|---|---|
| | | | | | | | |
| **Composés de formule I** | | | | **Doses** | | | |
| **n** | **R** | **$R_n$** | **$R_1$** | **0,02 ng/ml** | **0,2 ng/ml** | **2 ng/ml** | **20 ng/ml** |
| 3 | H | H | F | 3 | -3 | -32 | -51 |
| 3 | H | H | H | 21 | 15 | 11 | 14 |
| 4 | H | H | F | 10 | 6 | -4 | -36 |
| 4 | H | H | H | 17 | 9 | 9 | 0 |
| 5 | H | H | H | 56 | 47 | 46 | 36 |
| 6 | H | H | F | 15 | 4 | 5 | -9 |
| 6 | H | H | H | | 33 | | |
| 9 | H | H | H | 9 | 10 | 6 | -22 |
| 10 | H | H | F | 29 | 16 | 0 | -19 |
| 10 | H | H | H | 11 | 4 | -20 | -34 |
| 13 | H | H | F | 36 | 28 | 14 | -9 |
| 13 | H | H | H | 3 | 13 | -1 | -10 |
| 14 | H | H | F | 6 | -17 | -26 | -42 |
| Pré-incubation : 3h<br>Nombre d'essais = 3 pour tous les composés<br>Nombre d'essais = 18 pour la vit E | | | | | | | |

*In vitro,* à l'échelle cellulaire, un stress exogène par le $H_2O_2$ /$Fe^{2+}$-$Fe^{3+}$ est capable d'induire une production d'EAO intracellulaires détectés à l'aide de la sonde fluorescente.

En conclusion, les dérivés d'hydroxy acides gras insaturés à courtes chaînes (C9 et C10) et non fluorés semblent être plus particulièrement actifs dans la protection cellulaire anti-radicalaire.

Etude de l'effet anti-radicalaire. Analyse de la peroxydation lipidique

Par ailleurs, durant le métabolisme cellulaire normal, lors d'exposition occasionnelle de la peau à des agents stressants ou au cours de pathologies dermatologiques, des espèces oxygénées réactives encore appelées Radicaux Libres Oxygénés (RLO) sont générées (Y. M. W. Janssen *et al,* 1993). Ces RLO, décrits comme des métabolites très réactifs, jouent un rôle important dans bon nombre des processus tels que l'inflammation, le vieillissement et la promotion tumorale.

Les RLO sont considérés comme les « seconds messagers » dans la signalisation cellulaire du stress oxydatif et donc comme les médiateurs précoces de l'inflammation (A. Van Der Vliet and A. Bast, 1992).

Leur surproduction induit d'importants dommages au sein de la cellule. Certains constituants cellulaires sont

alors les cibles majeures d'un tel stress oxydatif : les composants lipidiques de la membrane plasmique (lipoperoxydation), les protéines (dénaturation et dégradation) et le matériel génétique ou ADN (mutations) peuvent être altérés. Les cellules sont capables de limiter ces dommages oxydatifs grâce à différents systèmes de défense antiradicalaire (antioxydants enzymatiques et non enzymatiques) (B. P. Yu, 1994 ; H. Steiling *et al,* 1999).

Cependant, dans certaines conditions, les RLO sont produits en quantité telle que l'activité antioxydante cellulaire est insuffisante ; ces RLO deviennent alors des facteurs inducteurs de pathologies inflammatoires et du vieillissement tissulaire (Y. Miyachi *et al,* 1986 ; M. Kress *et al,* 1995).

Afin de valoriser l'activité anti-radicalaire des différents dérivés hydroxylés selon l'invention, nous avons analysé leur pouvoir protecteur vis à vis de l'altération des membranes cellulaires induite par un stress oxydatif (chimique), en comparaison avec un antioxydant de référence, la vitamine E.

La membrane plasmique constitue la principale et la première cible des RLO et, étant riche en lipides, elle est le site d'une peroxydation accrue (A. W. Girotti, 1985). Les peroxydes générés au cours de cette oxydation lipidique sont aussi très réactifs et capables de dégrader le matériel protéique et génomique.

Pour évaluer l'altération membranaire, nous avons mesuré la peroxydation lipidique par un dosage *in vitro* des complexes entre les produits d'oxydation lipidique et l'acide thiobarbiturique. Ces complexes sont appelés TBARS (pour Thiobarbituric Acid Reactive Substances) et donnent le nom au test : Test des TBARS.

Afin de mimer un stress oxydatif chimique, nous avons traité la lignée de fibroblastes, L929, par un complexe composé de peroxyde d'hydrogène ($H_2O_2$) et de fer ($Fe^{2+}/Fe^{3+}$) reconstituant ainsi la réaction de Fenton, source de RLO et plus particulièrement de radical hydroxyle (OH°) (D.A Vessey *et al,* 1992):

$$H_2O_2 + Fe^{2+} \rightarrow OH° + OH^- + Fe^{3+}.$$

Les produits ont été évalués sur la lignée de fibroblastes murins L929. Les cellules sont prétraitées avec les différentes concentrations de produits pendant 16 heures et sont ensuite stimulées avec le complexe $H_2O_2$-$Fe^{2+}/Fe^{3+}$ (200μM-1mM) pendant 1 heure.
Solutions mères : 100 mg/ml, éthanol, 4°C.
Solutions finales : 0,02 ng/ml.

La peroxydation des lipides membranaires est analysée en mesurant les TBARS (Protocole Réf. PLN°2, selon Morlière *et al,* 1991).

*Principe du test :*

En milieu acide, à 95°C, se forment des complexes, notés TBARS pour Thio Barbituric Acid Reactive Substance, entre les produits d'oxydation lipidiques (malondialdéhyde ou MDA) et l'acide thiobarbiturique (TBA) qui peuvent être dosés en fluorescence par rapport à une gamme étalon avec le MDA. Le dosage des TBARS est alors exprimé en pmole/μg de protéines. Les protéines et TBARS sont dosés dans le milieu intracellulaire.

*Calcul du pourcentage de protection des membranes cellulaires :*

A partir du calcul des TBARS en pmole/μg protéines, nous avons calculé l'efficacité protectrice des différents produits contre l'oxydation des lipides membranaires.

$$\% \text{ de protection : } \frac{[\text{TBARS Contrôle}] - [\text{TBARS (+produits)}]}{\text{TBARS Contrôle}} \times 100$$

Quatre expériences indépendantes ont été réalisées. Durant ces expériences, divers composés ont été évalués (le test ne permet pas d'évaluer plus de 10 molécules en même temps). Les composés évalués plusieurs fois ont été choisis en fonction des résultats obtenus dans l'autre test mesurant également une activité anti-radicalaire (test de dosage des espèces actives de l'oxygène, EAO).

Le modèle utilisé dans cette expérience (réaction de Fenton) induit une peroxydation lipidique importante dans les fibroblastes L929. Cette décharge massive de radical hydroxyle OH° génère donc un stress oxydatif au niveau cellulaire et notamment au niveau des membranes. Cependant, dans ce type de réaction oxydative, les produits issus de la peroxydation lipidique sont internalisés dans les cellules et les TBARS sont alors dosés dans le milieu intracellulaire.

La vitamine E à 400μg/ml, diminue la peroxydation lipidique induite par le complexe $H_2O_2$-$Fe^{2+}/Fe^{3+}$, et protège très efficacement les membranes cellulaires.

Dans le tableau II, sont présentés les résultats des 4 expériences.

Il apparaît clairement que ces dérivés hydroxylés ont une activité antioxydante stable et reproductible. Il s'agit particulièrement de dérivés à chaîne courte de carbones.

Tableau II: Protection des lipides membranaires par les différents dérivés hydroxylés

| | | | | **% protection des lipides membranaires** | | |
|---|---|---|---|---|---|---|
| **Molécules testées** | | | | Nbre Expérience | Moyenne | Ecart type |
| **Vit E 400μg/ml** | | | | 4 | 44,66 | 25,85 |
| **Composés de formule I** | | | | | | |
| **N** | **R** | **$R_n$** | **$R_1$** | | | |
| 6 | H | H | H | 3 | 52,91 | 8,81 |
| 6 | H | H | F | 3 | 40,65 | 16,83 |
| 4 | H | H | F | 3 | 36,54 | 41,69 |
| 13 | H | H | H | 1 | 22,10 | |
| 3 | H | H | F | 1 | 38,14 | |
| 13 | H | H | F | 3 | 7,39 | 16,23 |
| 10 | H | H | H | 1 | 27,18 | |
| 4 | H | $CH_3$ | H | 2 | 57,75 | 3,15 |
| 10 | H | H | F | 3 | 13,13 | 13,37 |
| 14 | H | H | F | 1 | 35,30 | |
| 4 | H | H | H | 2 | 37,88 | 26,28 |
| 9 | H | H | H | 3 | 8,72 | 48,78 |
| 3 | H | H | H | 2 | 15,7 | 29,93 |

Le modèle *in vitro* présenté dans cette étude reflète les conséquences dues à un stress oxydatif majeur sur la principale cible cellulaire qu'est la membrane plasmique. Ainsi le dosage de la peroxydation lipidique constitue un bon marqueur du stress oxydatif et permet l'évaluation de l'action antioxydante, vis à vis du radical hydroxyle, de principes actifs au niveau de la membrane cellulaire.

La Vitamine E, molécule antioxydante, permet la validation de ce modèle et protège efficacement les membranes cellulaires du stress oxydatif.

Différenciation kératinocytaire

Enfin, l'épiderme participe à la fonction principale de la peau, qui est de résister à l'environnement et de s'en protéger. C'est un épithélium stratifié comifié squameux en perpétuel renouvellement. L'homéostasie et la régénération tissulaires reposent sur les cellules kératinocytaires, les cellules de soutien, les facteurs solubles, la proximité cellulaire favorisant les interactions des cellules entre elles et avec les matrices extracellulaires.

Le stratum cornéum, stade ultime de la différenciation épidermique, résulte de trois processus majeurs : formation des filaments de kératine, "cornification" du kératinocyte et formation du ciment lipidique intercellulaire organisé en structures lamellaires.

Au sein de l'épiderme, la composition et la nature des lipides varient en fonction de l'état de différenciation dans lequel se trouvent les kératinocytes. Ainsi, lors du passage des assises basales aux cellules de la couche cornée, on observe une réduction importante des phospholipides qui, dans les couches vivantes sont responsables de l'intégrité des membranes plasmiques; parallèlement, on note une augmentation des lipides neutres (acides gras libres et triglycérides) et des stérols (cholestérol), ainsi qu'une très forte augmentation des sphingolipides et notamment des céramides. Au niveau de la couche cornée, sont retrouvés 40 à 50 % de sphingolipides, 20 à 27 % de cholestérol et 9 à 26 % d'acides gras libres. Les céramides interviennent de manière covalente avec les résidus glutamiques de l'involucrine, précurseur de l'enveloppe cornée. Les acides gras, grâce à leur caractère hydrophobe, participent au contrôle de l'imperméabilité cutanée, le cholestérol intervenant dans la fluidité membranaire.

De nombreuses protéines spécifiques de la couche cornée ou stratum comeum, (couche conférant la fonction barrière à l'épiderme), sont produites au niveau de la couche granuleuse composée des derniers kératinocytes à présenter une activité transcriptionnelle et traductionnelle avant la lyse nucléaire qui accompagne la cornification.

Le programme de différenciation des kératinocytes est majoritairement axé sur l'évolution de protéines de structure que sont les kératines et qui contribuent à l'intégrité architecturale de l'épiderme. Leur expression varie en fonction du degré de maturation des cellules épidermiques. Dans les couches suprabasales apparaissent la kératine 1 basique et la kératine 10 acide, dites kératines de différenciation terminale.

Au voisinage de la couche cornée, les kératines interagissent avec des protéines riches en histidine pour former un mélange relativement homogène qui remplit les cornéocytes. Ces protéines dérivent d'un précurseur, la profilaggrine qui est une grosse molécule ([PM]>450 kDa) stockée au niveau de la couche granuleuse. C'est une protéine dont les domaines N et C terminaux lient le calcium et dont la déphosphorylation et une protéolyse partielle donnent la filaggrine. Celle-ci catalyse la formation de ponts disulfures entre les filaments de kératines et contribue à leur intégration au sein de la matrice intracornéocytaire. D'autre part elle est la source, par protéolyse, d'un ensemble d'acides aminés et de dérivés peptidiques qui confèrent à la couche cornée certaines capacités de rétention hydrique.

Dans la couche cornée, les cornéocytes contiennent essentiellement des filaments de kératine insérés dans une matrice dense, le tout entouré par une paroi protéique épaisse et résistante : l'enveloppe cornée. Les protéines précurseur de cette structure, apposées à la face interne de la membrane plasmique, sont synthétisées par les kératinocytes à partir de la couche épineuse, mais leurs pontages - sous l'action d'enzymes, les transglutaminases - s'effectue entre les couches épineuses et granuleuses. De nombreuses protéines cytosoliques, associées à la fraction membranaire des kératinocytes ou stockées dans les grains de kératohyaline, participent à l'élaboration de l'enveloppe cornée. On peut ainsi distinguer l'involucrine (68 kDa), riche en glutamine et en lysine.

L'imperméabilité de la couche cornée est due, en majeure partie, à un ciment intercellulaire lipidique hydrophobe qui réunit les cornéocytes. Le contenu lipidique de l'épiderme est le résultat final de la lipogenèse kératinocytaire et sébacée. Dans l'épiderme normal, un mélange de lipides neutres et polaires prédomine dans les couches profondes pour être progressivement remplacé par un contenu plus apolaire, incluant des céramides. De nombreuses protéines participent à cette lipogénèse et donc à la synthèse des céramides. Parmi elles, la protéine DES2 (Sphingolipid C4-hydroxylase / delta-4 desaturase) a été mise en évidence : elle possède une activité dihydrocéramide hydroxylase qui intervient dans la synthèse des phytocéramides et son expression est induite avec la différenciation (MIZUTANI Y. *et Al,* 2004). Les lipides synthétisés par les kératinocytes sont acheminés à la surface par les corps lamellaires, les kératinosomes. Ils sont sécrétés dans les espaces intercellulaires et s'organisent en feuillets continus alignés parallèlement aux membranes cellulaires des cornéocytes. Cette réorganisation lipidique survenant au cours de la différenciation épidermique fait intervenir un certain nombre de transporteurs lipidiques de la famille des transporteurs ABC (Adenosine Triphosphate Binding Cassette transporter). Parmi ces transporteurs, certains voient leur expression induite au cours de la différenciation kératinocytaire (ABCB1, ABCC1, ABCC3, ABCC4, et ABCG1), d'autres voient leur expression réprimée (ABCB9 et ABCD1) et les autres ne subissent aucune régulation. Le rôle des transporteurs dont l'expression est induite a été mis en évidence : ABCG1 est par exemple impliqué dans la translocation du cholestérol dans les kératinocytes en différenciation. L'expression de certains membres d'une autre famille de transporteurs, celle des FATP (Fatty Acid Transport Protein), tels que FATP1, FATP3, FATP4 et FATP5 est induite au cours de la différenciation kératinocytaire. Ces transporteurs seraient impliqués dans la réorganisation du pool de lipides neutres (KIELAR *et Al,* 2003).

Les kératinocytes humains normaux (NHK) prolifèrent et se différencient *in vitro,* formant des couches stratifiées mimant l'organisation basale de la peau. Des concentrations extracellulaires en calcium supérieures à 0.1 mM initient une cascade d'événements génomiques et non génomiques qui conduit les kératinocytes d'une phase de prolifération vers une phase de différenciation. La différenciation des kératinocytes peut alors être évaluée par l'étude de l'expression de plusieurs marqueurs tels que les kératines 1 et 10, la transglutaminase 1, la désaturase DES2, l'involucrine, les transporteurs FATP4 et ABCG1. Nous avons ainsi évalué dans un modèle de culture *in vitro* l'effet de certains acides hydroxyalcène carboxyliques.

Le plan de l'étude peut être schématisé comme suit : différenciation des NHK in vitro et prélèvements pour analyse

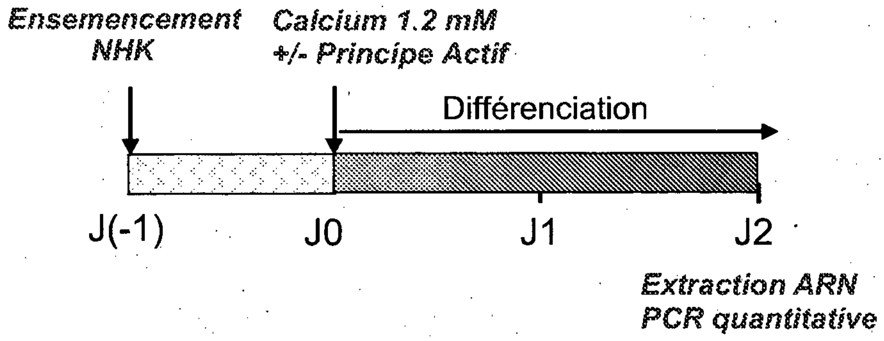


Conditions de culture et de prélèvement

Les Kératinocytes Humains Normaux (NHK) sont isolés à partir d'explants de peau. Ils sont cultivés dans un milieu KSFM (Gibco) à faible teneur en calcium (0.1 mM) contenant 25 μg/mL de BPE (Bovin Pituitary Extract) et 1.5 ng/mL d'EGF (Epithelium Growth Factor). Vingt-quatre heures après ensemencement, la différenciation est induite par l'ajout de calcium (concentration finale 1.2 mM). Les principes actifs sont ajoutés ou non en même temps que le calcium à des concentrations de 100 et 1 μg/mL afin de déterminer s'ils ont un effet de potentialisation de la différenciation. Les cellules sont alors analysées 48h00 après l'ajout du calcium et des produits.

Extraction des ARNs et RT-PCR en temps réel

Les ARNs totaux sont extraits à froid à partir des kératinocytes.

Après transcription inverse des ARNm en cDNA, les PCRs sont réalisées en plaque 96 puits (appareil à PCR quantitative iCycler, BioRad). L'expression des transcrits de la transglutaminase, de la désaturase (DES2), ABCG1 et FATP4 est normalisée par rapport à l'expression de gènes de référence (GAPDH, UBC, HPRT). Les niveaux d'expression des échantillons traités par le calcium et le principe actif sont comparés aux niveaux d'expression des échantillons traités uniquement par le calcium.

Les résultats des effets des produits à 1 μg/mL sont consignés dans les tableaux ci-après :

| **Composés de formule I** | | | | **Facteur d'induction vs contrôle (CaCl2 1.2 mM)** | **TG1** | **DES2** | **ABCG1** | **FATP4** |
|---|---|---|---|---|---|---|---|---|
| **n** | **R** | **$R_n$** | **$R_1$** | Vit. D3 1μM | 1.5 | 2.0 | 2.0 | 1.1 |
| 3 | H | H | F | | 1.5 | 1.7 | 1.5 | 1.2 |
| 3 | H | H | H | | 1.2 | 1.6 | 1.5 | 1.4 |
| 4 | H | H | F | | 1.6 | 2.2 | 2.6 | 1.3 |
| 4 | H | H | H | | 1.2 | 1.0 | 1.2 | 1.0 |
| 5 | H | H | H | | 1.5 | 1.8 | 2.7 | 1.0 |
| 6 | H | H | F | | 1.2 | 1.3 | 1.3 | 1.0 |
| 6 | H | H | H | | 1.1 | 1.7 | 1.9 | 1.2 |
| 9 | H | H | H | | 1.4 | 1.8 | 1.5 | 1.2 |
| 10 | H | H | F | | 1.4 | 1.6 | 1.4 | 1.0 |
| 10 | H | H | H | | 1.5 | 2.0 | 1.4 | 1.2 |
| 13 | H | H | F | | 4.2 | 9.0 | 4.8 | 2.2 |
| 13 | H | H | H | | 1.4 | 3.0 | 3.0 | 1.4 |
| 14 | H | H | F | | 1.7 | 2.8 | 4.0 | 1.0 |

A la dose de 1 μg/mL, l'ensemble des produits a un effet modéré sur l'expression des marqueurs de différenciation.

Effets des produits à 100 μg/mL

| Composés de formule I | | | | Facteur d'induction vs contrôle (CaCl2 1.2 mM) | TG1 | DES2 | ABCG1 | FATP4 |
|---|---|---|---|---|---|---|---|---|
| n | R | $R_n$ | $R_1$ | Vit. D3 10 μM | 8.3 | 11.0 | 3.1 | 1.3 |
| 3 | H | H | F | | 0.9 | 1.9 | 1.2 | 1.2 |
| 3 | H | H | H | | 2.6 | 2.4 | 4.0 | 1.2 |
| 4 | H | H | F | | 1.7 | 3.2 | 2.6 | 1.4 |
| 4 | H | H | H | | 1.7 | 2.2 | 2.5 | 1.4 |
| 6 | H | H | F | | 7.8 | 10.3 | 6.3 | 1.9 |
| 6 | H | H | H | | 12.3 | 11.5 | 6.6 | 2.5 |
| 9 | H | H | H | | 14.8 | 51.7 | 13.8 | 4.5 |
| 10 | H | H | F | | 3.5 | 16.0 | 4.6 | 2.3 |
| 10 | H | H | H | | 31.4 | 125.8 | 25.5 | 5.9 |

La vitamine D3 à 10 μM induit l'expression des transcrits de la transglutaminase 1, DES2, et ABCG1 d'un facteur 8.3, 11.1 et 3.1 respectivement, par rapport au témoin traité uniquement par le calcium. L'expression de FATP4 par contre n'est pas modulée par le traitement vitamine D3.

L'effet de potentialisation de la différenciation a donc été démontré à propos des dérivés d'hydroxy-acides gras insaturés selon l'invention.

<u>Effet anti inflammatoire analysé au niveau des médiateurs lipidiques de l'inflammation</u>

Le kératinocyte, cellule la plus représentée au niveau de l'épiderme, libère, en réponse à de nombreux facteurs extra-cellulaires présents dans son environnement, des médiateurs biologiquement actifs, notamment des prostaglandines et les leucotrienes qui jouent un rôle important dans l'initiation et la modulation des réactions inflammatoires cutanées et qui interviennent également dans la régulation de la réponse immune. La prostaglandine PG6KF1alpha, est un des métabolites majeurs produits par le kératinocyte stimulé, et représentatif de la modulation de la production des métabolites du métabolisme de l'acide arachidonique issus de la voie de la cyclo-oxygénase

PROTOCOLE :

La suspension de kératinocytes dans le DMEM 10% SVF est distribuée dans des plaques 6 puits ($1,2.10^6$ cellules/puits), et incubée pendant 16 heures à 37°C dans une atmosphère à 5% $CO_2$. Les kératinocytes sont alors rincés avec du PBS pour éliminer les cellules non-adhérentes puis exposés aux produits à tester inclus dans du DMEM sans SVF (qui pourrait interférer dans le dosage).

La concentration testée en culture est de 3μg/ml. Elle a été retenue après un test préliminaire d'évaluation de la cytotoxicité (rouge neutre) et n'est pas cytotoxique.

Pour chaque traitement, 3 puits sont réalisés. Les cellules sont pré-incubées pendant 60 mn avec les produits à tester puis un agent stimulant de la cascade de l'acide arachidonique, le ionophore calcique, est ajouté pendant 5 heures : le ionophore calcique A23187 est utilisé à la concentration 1μM.

Après ces 5 heures de culture, les milieux de culture de chacun des puits sont récupérés, centrifugés à 3000 tr/mn et conservés à -80°C.

La production de prostaglandine 6KF1α pour chacun des essais est mesurée par Kit Elisa EUROMEDEX.

RESULTATS:

Les résultats sont exprimés en pourcentage d'activité / contrôle stimulé.

| Composés de formule I | | | | % d'activité inhibitrice des molécules évaluées sur la production de PG6KF1α |
|---|---|---|---|---|
| | | | | Inflammation |
| n | R | $R_n$ | $R_1$ | 3μg/ml |
| 3 | H | H | H | 29 |
| 4 | H | H | F | 30 |
| 4 | H | H | H | |
| 4 | H | $CH_3$ | H | |
| 8 | H | H | H | 32 |
| 8 | H | H | F | 44 |
| 9 | H | H | H | 31 |
| 10 | H | H | F | 18 |
| 10 | H | H | H | Inactif |
| 13 | H | H | F | 14 |
| 13 | H | H | H | 25 |

Propriété anti inflammatoire : Inhibition de la synthèse d'une cytokine pro-inflammatoire IL8

La fonction barrière de la peau permet d'assurer une protection vis à vis de l'environnement extérieur, et les kératinocytes de l'épiderme peuvent directement répondre à une large variété d'agents irritants ou allergènes et participer activement aux processus inflammatoires et immunitaires cutanés, en particulier à travers la génération de cytokines proinflammatoires, médiateurs d'origine protéique. Parmi ces molécules biologiquement actives, l'IL1α (Interleukin 1 α) et le TNFα (Tumor Necrosis Factor α) sont considérées comme des cytokines primaires, leur libération étant suffisante pour induire l'inflammation en vertu de leur induction de molécules d'adhésion au niveau des cellules endothéliales et de leur induction de facteurs chimiotactiques tels que les chemokines. Le système des chemokines contrôle le trafic leucocytaire pendant la réponse inflammatoire et est nécessaire aux interactions des réponses immunes innées et adaptatives.

Dans l'étude présente nous nous sommes plus spécifiquement intéressés à la chemokine - Interleukine 8 - qui est fortement impliquée dans l'amplification de la réponse inflammatoire et dont la fonction principale est de recruter et activer les polynucléaires neutrophiles notamment en stimulant leur libération de molécules pro-inflammatoires.

Dans cette étude, réalisée sur des plaques 96 puits, nous avons évalué l'activité des acides hydroxy-alcènes, sur la production d'interleukine 8 induite au niveau du kératinocyte par le phorbol ester PMA et le ionophore calcique A23187.

PROTOCOLE :

La suspension de kératinocytes dans le KSFM complémenté est distribuée dans des plaques 96 puits ($3.10^4$ cellules/puits), et incubée pendant 16 heures à 37°C dans une atmosphère à 5% $CO_2$. Les kératinocytes sont alors rincés avec du PBS pour éliminer les cellules non-adhérentes puis exposés aux produits à tester inclus dans du KSFM non complémenté (qui pourrait interférer dans le dosage).

La concentration testée en culture est de 3μg/ml. Elle a été retenue après un test préliminaire d'évaluation de la cytotoxicité (rouge neutre) et n'est pas cytotoxiques.

Pour chaque traitement, 3 puits sont réalisés. Les cellules sont pré-incubées pendant 60 mn avec les produits à tester puis stimulées, parallèlement à des contrôles négatifs sans stimulant : Phorbol Myristate Acetate (PMA) 1μM + Ionophore calcique (A23187) 0,1μM.

Incubation pendant 6 heures à 37°C en atmosphère humide d'air contenant 5% de $CO_2$.

Les milieux de culture de chacun des puits sont récupérés, centrifugés à 3000 tr/mn et conservés à -80°C.

Dosage des cytokines : L'IL8 est dosée par une méthode immunoenzymatique en kit ELISA (Immunotech).

RESULTATS :

Les résultats sont exprimés en pourcentage d'activité / contrôle stimulé.

| Composés de formule I | | | | % d'activité inhibitrice des molécules évaluées sur la production de PG6KF1$\alpha$ |
|---|---|---|---|---|
| | | | | Inflammation |
| n | R | $R_n$ | $R_1$ | 3$\mu$g/ml |
| 3 | H | H | H | 50 |
| 4 | H | H | F | Inactif |
| 4 | H | H | H | 15 |
| 4 | H | $CH_3$ | H | Inactif |
| 6 | H | H | H | Non déterminé |
| 8 | H | H | H | Inactif |
| 8 | H | H | F | Inactif |
| 9 | H | H | H | 16 |
| 10 | H | H | F | 30 |
| 10 | H | H | H | 30 |
| 13 | H | H | F | 107 |
| 13 | H | H | H | 62 |
| 14 | H | H | H | Non actif |

Les composés selon l'invention ont été évalués en terme d'activité anti-inflammatoire sur la libération d'interleukine 8 par les kératinocytes humains NHK stimulés par phorbol ester PMA + le ionophore calcique A23187.

Trois expérimentations indépendantes ont été réalisées : la synthèse des données obtenues permet de mettre en évidence les potentialités anti-inflammatoires en particulier des 2 composés suivants :

- n = 13, R = Rn = H, $R_1$ = F
- n = 13, R = $R_n$ = $R_1$ = H

Pour la concentration évaluée pour chacune des molécules et après calcul des Doses Efficaces 50, nos résultats montrent ce qui suit :

| Composés de formule I | | | | % Inhibition à 3$\mu$g/ml | DE 50 |
|---|---|---|---|---|---|
| n | R | $R_n$ | $R_1$ | | |
| 13 | H | H | F | 107 | <3$\mu$g/ml |
| 13 | H | H | H | 62 | <3$\mu$g/ml |

Inflammation et prurit : Analyse du flux calcique suite à la stimulation des récepteurs PAR2

Le récepteur « Protease-Activated Receptor-2 (PAR-2) » est associé à la physio-pathologie de plusieurs maladies impliquant des réponses inflammatoires.

Le PAR-2 est exprimé par les différents types cellulaires de la peau : kératinocytes, cellules myoépithéliales des glandes sudoripares, follicule pileux, cellules dentritiques-like du derme et les cellules endothéliales de la lamina propria et du derme (Steinhoff et al., 1999 ; Santulli et al., 1995). Les mélanocytes n'expriment pas ce récepteur (Seiberg et al., 2000) bien que le PAR-2 joue un rôle important dans la pigmentation en favorisant le transfert de mélanine des mélanocytes vers les kératinocytes (Sharlow et al., 2000).

Les sérine-protéases générées par l'épiderme exercent des effets chimiotactiques induisant le recrutement

des leucocytes dans la peau. Elles sont également impliquées dans la régulation de l'homéostasie, de la mitogenèse et de la différenciation épidermiques et modulent la fonction barrière de la peau. De plus, les sérine-protéases contribuent à la physiopathologie de maladies cutanées liées à l'inflammation, la défense de l'hôte, la cancérogenèse, la fibrose et la stimulation nerveuse.

Les propriétés physiologiques et physiopathologiques cutanées des sérine protéases seraient en partie liées aux récepteurs PARs. En effet, les récepteurs PAR-2 sont sur-exprimés dans l'épiderme, le derme et les vaisseaux des maladies inflammatoires de la peau comme la dermatite atopique, le lichen plan et le psoriasis (Steinhoff et al., 1999). Les récepteurs PAR-2 joueraient également un rôle dans le développement du prurit chez des patients atteints de dermatite atopique (Steinhoff et al., 2003).

L'activation de PAR-2 par une protéase de type trypsine induit la production d'IL-8 à partir de kératinocytes (HaCaT) (Hou et al., 1998). Plus récemment, il a été démontré que l'IL-8, chémokine chimio-attractive pour les leucocytes, permettrait l'infiltration de neutrophiles dans l'épiderme de patients atteints de Psoriasis vulgaris (Iwakiri et al., 2004).

La signalisation intracellulaire du récepteur PAR-2 est sous-tendue pour partie par une mobilisation de calcium intra et extra-cellulaire.

Nous nous sommes donc proposés d'évaluer l'activité anti-PAR-2 des dérivés hydroxy acides de formule I, sur l'influx de calcium intracellulaire induit après stimulation spécifique par des récepteurs PAR-2 la trypsine présents sur les kératinocytes humains issus d'une lignée (HaCaT).

Cette technique est basée sur l'utilisation d'une sonde fluorescente estérifiée par un groupement AM, facilitant sa pénétration par diffusion passive dans la cellule. La sonde utilisée est le Fluo-4/AM. Seule la forme désestérifiée et liée aux ions calcium est excitable en fluorescence (à 485 nm) et émet à 535 nm.

Pourcentage d'inhibition du flux calcique induit par la trypsine

| | | % Stimulation | Ecartype | % Inhibition |
|---|---|---|---|---|
| **Sans sonde** | | 3,82 | 0,79 | - |
| **Trypsine 10nM** | | 13,67 | 3,14 | - |
| **STI 1µM** | | 8,39 | 2,85 | 54 |
| **$R = R_n = H$ $R_1 = F$ $n = 13$** | **0,1µg/ml** | 13,78 | 0,82 | -1 |
| | **0,3µg/ml** | 13,06 | 1,47 | 6 |
| | **1µg/ml** | 12,74 | 1,25 | 9 |
| | **3µg/ml** | 12,97 | 0,92 | 7 |
| | **10µg/ml** | 9,55 | 1,96 | 42 |
| **$R = R_n = R_1 = H$ $n=10$** | **10µg/ml** | 15,33 | 3,00 | -17 |
| | **30µg/ml** | 14,10 | 3,42 | -4 |
| | **100µg/ml** | 9,84 | 3,26 | 39 |
| **$R = R_n = R_1 = H$ $n = 14$** | **10µg/ml** | 14,68 | 2,56 | -10 |
| | **30µg/ml** | 12,90 | 3,58 | 8 |
| | **100µg/ml** | 11,37 | 2,62 | 23 |

Stimulation par la trypsine

Sur les kératinocytes issus d'une lignée (HaCaT) :

les dérivés hydroxy-acides de formule générale (I), mentionnés dans le tableau ci-dessus présentent une activité sur l'inhibition du flux calcique induit par la trypsine, à une concentration de 10µg/ml pour le premier, et à une concentration de 100µg/ml pour les deux composés suivants.

In vitro, à l'échelle cellulaire, la stimulation spécifique des récepteurs PAR-2 par la trypsine conduit à une mobilisation de calcium intra et extracellulaire détectée à l'aide de la sonde fluorescente.

Dans les conditions expérimentales et aux concentrations testées, les composés de formule générale (I) mentionnés dans le tableau ci-dessus modulent de façon significative l'activité anti-PAR-2.

Effet des dérivés d'hydroxy-acides gras sur la synthèse de mélanine in vitro

Les mélanocytes sont des cellules d'aspect étoilé, présentes en minorité dans la couche basale de l'épiderme. Leur fonction principale est d'assurer la mélanogenèse, processus par lequel la mélanine est synthétisée dans des organites spécialisés, les mélanosomes, puis transportée et distribuée aux kératinocytés voisins via leurs prolongements dendritiques. Ce contact avec les kératinocytes permet la pigmentation cutanée, mécanisme de protection de l'épiderme contre les effets mutagènes des rayons ultraviolets. Chaque mélanocyte est en relation avec environ trente six kératinocytes, formant ainsi une « unité épidermique de mélanisation ».

La mélanogenèse consiste en une série de réactions enzymatiques et spontanées, dont le précurseur est la tyrosine. Trois enzymes principales participent à ce processus : la tyrosinase, et les tyrosinase-related protein 1 et 2 (TRP 1 et 2) (1). La tyrosinase catalyse la transformation de la tyrosine en dopaquinone. A partir de là, deux voies de synthèse sont alors possibles : l'eumélanogenèse et la phéomélonogenèse. La conversion de dopaquinone en eumélanine se fait par une série de réactions successives d'oxydations faisant intervenir TRP-1 et TRP-2. L'eumélanine correspond au pigment brun noir, à faible teneur en soufre, et assure le pouvoir photoprotecteur. Dans la phéomélanogenèse, des molécules à forte teneur en soufre s'incorporent à la dopaquinone pour donner la phéomélanine, de couleur jaune-orangé, présente dans les peaux des sujets roux.

Le stimulus physiologique de la synthèse de mélanine est le soleil, ce qui entraîne une augmentation du nombre des mélanocytes, une néosynthèse de mélanine, et des modifications morphologiques des mélanocytes, associant un accroissement de leur dendricité à une augmentation du transfert des mélanosomes aux kératinocytes. Au niveau moléculaire, l'exposition au soleil stimule la synthèse et la sécrétion d'alpha melanocyte stimulating hormone. L'$\alpha$-MSH augmente la concentration intramélanocytaire en AMPc, activant le facteur de transcription, Mitf, qui stimule à son tour l'activité transcriptionnelle des gènes codant pour la tyrosinase, TRP-1 et TRP-2 .

Certaines molécules exogènes sont également connues pour réguler négativement la mélanogenèse. L'hydroquinone inhibe la synthèse de mélanine en se présentant comme substrat de la tyrosinase afin de détourner son activité.

L'effet modulateur des dérivés d'hydroxy acides gras insaturés, sur la mélanogénèse a été analysé. Pour cela une mesure de la synthèse de mélanine par dosage colorimétrique est effectuée sur une lignée cellulaire de mélanomes murins : la lignée B16-F10 .

L'effet des produits est investigué en situation basale et après stimulation de la mélanogénèse par $\alpha$ MSH pour déterminer le pouvoir dépigmentant .

Dosage de la mélanine

Le taux de mélanine extracellulaire et intracellulaire est alors mesuré par spectrophotométrie à une longueur d'onde de 405 nm selon le protocole (Anob et al, 1999). La quantité de pigment est déterminée grâce à une gamme étalon de mélanine et l'analyse sur le logiciel Microwin (Berthold Biotechnologies). Un dosage de protéines totales est effectué pour les échantillons de mélanine intracellulaire par la méthode BCA-Copper à 540nm. La gamme étalon est réalisée avec une protéine standard, la BSA (Serum Albumine Bovine).

RESULTATS

En situation basale l'alpha MSH à 1 $\mu$M augmente de 100% la production de mélanine par rapport aux cellules témoins.

Cette augmentation de mélanine est contrée par les agents dépigmentants tels l'hydroquinone à 1 $\mu$g/ml ou la vitamine C à 40 $\mu$g/ml, qui inhibent de 60% l'action de l'alpha MSH .

En situation basale à 30 $\mu$g/ml:

- Le composé de formule générale 1 avec n=10, $R=R_n=H$, $R_1=F$ inhibe de 30 à 45% la synthèse de mélanine alors qu'en présence d'alpha MSH, ce même composé à 30$\mu$g/ml entraîne une inhibition de 45 à 60 %.

- Le composé de formule générale 1 avec n=10, $R=R_1=R_n=H$ à 30$\mu$g /ml présente une activité plus faible que le composé précédent, 15% d'inhibition en situation basale alors qu'en présence d'alpha MSH le potentiel d'inhibition de ce composé à l'égard de cette substance mélanotrope, est similaire à celui du composé précédent c'est à dire de 45% à 60%.

A 10$\mu$g/ml l'activité inhibitrice de ces 2 acides sur l'induction de la mélanogénèse par alpha MSH est de l'ordre de 35%.

## Revendications

1. Dérivés d'hydroxy-acides gras insaturés répondant à la formule générale (I) :

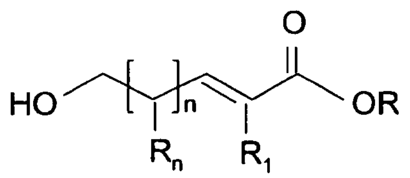

(I)

dans laquelle :

. $R_n$ représentent indépendamment les uns des autres H ou un groupe alkyle linéaire ou ramifié comprenant de 1 à 6 atomes de carbone et étant éventuellement substitué par un atome d'halogène en particulier de fluor,
. $R_1$ représente H, F, Cl, Br ou $CF_3$,
. R représente H, et
. $3 \leq n \leq 14$,

à la condition toutefois que, lorsque $n \neq 10$, l'un au moins des radicaux $R_1$ et $R_n$ ne représente pas l'hydrogène.

2. Dérivés d'hydroxy-acides gras insaturés de formule générale (I) selon la revendication 1, dans laquelle :

. $R_1$ représente F, Cl, Br ou $CF_3$.

3. Dérivés d'hydroxy-acides gras insaturés de formule générale (I) selon la revendication 1, dans laquelle :

. $6 \leq n \leq 14$.

4. Dérivés d'hydroxy-acides gras insaturés de formule générale (I) selon la revendication 1, dans laquelle :

. $R_1$ représente F, Cl, Br ou $CF_3$ ;
. $R_n$ = H.

5. Dérivés d'hydroxy-acides gras insaturés de formule générale (I) selon la revendication 1, dans laquelle :

. $R_1$ représente F, Cl, Br ou $CF_3$, et
. au moins l'un des radicaux $R_n$ représentant un groupe alkyle linéaire ou ramifié comprenant de 1 à 6 atomes de carbone et étant éventuellement substitué par un atome d'halogène en particulier de fluor.

6. Dérivés d'hydroxy-acides gras insaturés de formule générale (I), selon les revendications 1 ou 2, dans laquelle :

. $R_1$ = F,
. $R_n$ = H.

7. Dérivés d'hydroxy-acides gras insaturés de formule générale (I) selon la revendication 6, dans laquelle :

. $R_1$ = F,
. $R_n$ = H,
. n = 13.

8. Dérivés d'hydroxy-acides gras insaturés de formule générale (I) selon la revendication 2, dans laquelle :

. $R_1$ = F,
. $R_n$ = H,
. n = 6.

**9.** Dérivés d'hydroxy-acides gras insaturés de formule générale (I) selon la revendication 1, dans laquelle :

. $R = R_1 = H$,
. un seul des radicaux $R_n$ représente un groupe méthyle, les autres étant des atomes d'hydrogène, et
. $n = 4$.

**10.** Dérivés d'hydroxy-acides gras insaturés de formule générale (I) selon la revendication 1, dans laquelle :

. $R_1 = R_n = H$, et
. $n = 10$.

**11.** Dérivés d'hydroxy-acides gras insaturés de formule générale (I) selon la revendication 2, dans laquelle :

- $R_1 = F$,
. $R_n = H$, et
. $n = 10$.

**12.** Dérivés d'hydroxy-acides gras insaturés de formule générale (I) selon la revendication 2, dans laquelle :

. $R_1 = F$,
. $R_n = H$, et
. $n = 8$.

**13.** Compositions dermocosmétologiques comprenant un dérivé d'hydroxy-acide gras insaturé de formule générale (I) selon l'une des revendications 1 à 12, associé à un excipient dermatologiquement acceptable.

**14.** Utilisation d'un dérivé d'hydroxy-acide gras insaturé de formule générale (I) selon l'une des revendications 1 à 12, pour la fabrication d'une composition dermocosmétologique destinée au traitement des troubles de la pigmentation.

**15.** Utilisation d'un dérivé d'hydroxy-acide gras insaturé de formule générale (I) selon l'une des revendications 1 à 12, pour la fabrication d'une composition dermocosmétologique destinée au traitement du vieillissement cutané et des taches de vieillesse blanches ou brunes.

**16.** Utilisation d'un dérivé d'hydroxy-acide gras insaturé de formule générale (I) selon l'une des revendications 1 à 12, pour la fabrication d'une composition dermocosmétologique destinée à provoquer un éclaircissement de la peau.

**17.** Utilisation d'un dérivé d'hydroxy-acide gras insaturé répondant à la formule générale (I) :

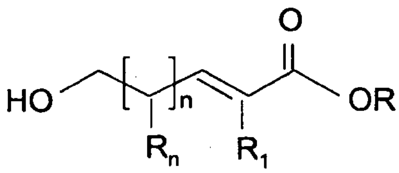

(I)

dans laquelle :

. $R_n$ représentent indépendamment les uns des autres H ou un groupe alkyle linéaire ou ramifié comprenant de 1 à 6 atomes de carbone et étant éventuellement substitué par un atome d'halogène en particulier de fluor,
. $R_1$ représente H, F, Cl, Br ou $CF_3$,
. R représente H ou un groupe alkyle linéaire ou ramifié comprenant de 1 à 6 atomes de carbone et étant éventuellement substitué par un atome d'halogène en particulier de fluor, et
. $3 \leq n \leq 14$,

pour la fabrication d'une composition dermocosmétologique anti-radicalaire, anti-inflammatoire, anti-purigineuse, et/ou destinée au traitement des troubles de la kératinisation, et/ou à améliorer la cicatrisation.

**18.** Utilisation d'un dérivé d'hydroxy-acide gras insaturé répondant à la formule générale (I) :

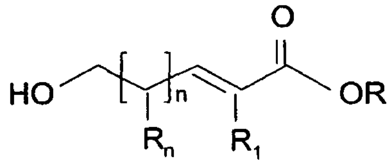

(I)

dans laquelle :

. $R_n$ représentent indépendamment les uns des autres H ou un groupe alkyle linéaire ou ramifié comprenant de 1 à 6 atomes de carbone et étant éventuellement substitué par un atome d'halogène en particulier de fluor,
. $R_1$ représente H, F, Cl, Br ou $CF_3$,
. R représente H ou un groupe alkyle linéaire ou ramifié comprenant de 1 à 6 atomes de carbone et étant éventuellement substitué par un atome d'halogène en particulier de fluor, et
. $3 \leq n \leq 14$,

pour la fabrication d'une composition dermocosmétologique destinée au traitement du psoriasis, du prurit et/ou de la dermite atopique.

**Patentansprüche**

**1.** Ungesättigte Fetthydroxysäurederivate entsprechend der allgemeinen Formel (I):

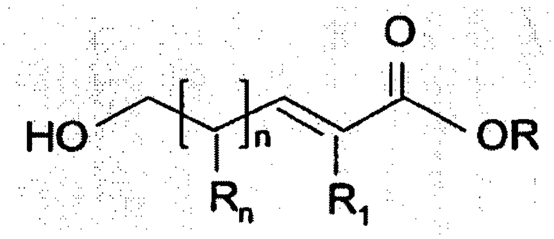

(I)

wobei Folgendes gilt:

• die $R_n$ stehen unabhängig voneinander für Folgendes: H oder eine geradkettige oder verzweigtkettige Alkylgruppe, die 1 bis 6 Kohlenstoffatome umfasst und die gegebenenfalls mit einem Halogenatom substituiert ist, insbesondere mit Fluor,
• $R_1$ steht für H, F, Cl, Br oder $CF_3$,
• R steht für H, und
• $3 \leq n \leq 14$,

wobei jedoch die Bedingung gilt, dass wenn $n \neq 10$, wenigstens eines der Radikale $R_1$ und $R_n$ nicht für Wasserstoff steht.

**2.** Ungesättigte Fetthydroxysäurederivate mit der allgemeinen Formel (I) nach Anspruch 1, wobei:

• $R_1$ für F, Cl, Br oder $CF_3$ steht.

**3.** Ungesättigte Fetthydroxysäurederivate mit der allgemeinen Formel (I) nach Anspruch 1, wobei:

• $6 \leq n \leq 14$.

**4.** Ungesättigte Fetthydroxysäurederivate mit der allgemeinen Formel (I) nach Anspruch 1, wobei:

- $R_1$ für F, Cl, Br oder $CF_3$ steht;
- $R_n$ = H.

**5.** Ungesättigte Fetthydroxysäurederivate mit der allgemeinen Formel (I) nach Anspruch 1, wobei:

- $R_1$ für F, Cl, Br oder $CF_3$ steht, und
- wenigstens eines der Radikale $R_n$ für eine geradkettige oder verzweigtkettige Alkylgruppe steht, die 1 bis 6 Kohlenstoffatome umfasst und die gegebenenfalls mit einem Halogenatom substituiert ist, insbesondere mit Fluor.

**6.** Ungesättigte Fetthydroxysäurederivate mit der allgemeinen Formel (I) nach Anspruch 1 oder 2, wobei:

- $R_1$ = F,
- $R_n$ = H.

**7.** Ungesättigte Fetthydroxysäurederivate mit der allgemeinen Formel (I) nach Anspruch 6, wobei:

- $R_1$ = F,
- $R_n$ = H,
- n = 13.

**8.** Ungesättigte Fetthydroxysäurederivate mit der allgemeinen Formel (I) nach Anspruch 2, wobei:

- $R_1$ = F,
- $R_n$ = H,
- n=6.

**9.** Ungesättigte Fetthydroxysäurederivate mit der allgemeinen Formel (I) nach Anspruch 1, wobei:

- R = $R_1$ = H,
- nur ein einziges der Radikale $R_n$ für eine Methylgruppe steht und die anderen Wasserstoffatome bezeichnen, und
- n=4.

**10.** Ungesättigte Fetthydroxysäurederivate mit der allgemeinen Formel (I) nach Anspruch 1, wobei:

- $R_1$ = $R_n$ = H, und
- n=10.

**11.** Ungesättigte Fetthydroxysäurederivate mit der allgemeinen Formel (I) nach Anspruch 2, wobei:

- $R_1$ = F,
- $R_n$ = H, und
- n=10.

**12.** Ungesättigte Fetthydroxysäurederivate mit der allgemeinen Formel (I) nach Anspruch 2, wobei:

- $R_1$ = F,
- $R_n$ = H, und
- n = 8.

**13.** Dermakosmetische Zusammensetzung, die ein ungesättigtes Fetthydroxysäurederivat mit der allgemeinen Formel (I) nach einem der Ansprüche 1 bis 12 umfasst und die mit einem dermatologisch verträglichen Exzipienten verbunden ist.

**14.** Verwendung eines ungesättigten Fetthydroxysäurederivats mit der allgemeinen Formel (I) nach einem der Ansprüche 1 bis 12 zur Herstellung einer dermakosmetischen Zusammensetzung, die für die Behandlung von Pigmentstörungen vorgesehen ist.

**15.** Verwendung eines ungesättigten Fetthydroxysäurederivats mit der allgemeinen Formel (I) nach einem der Ansprüche 1 bis 12 zur Herstellung einer dermakosmetischen Zusammensetzung, die für die Behandlung der Hautalterung und von weißen oder braunen Altersflecken vorgesehen ist.

**16.** Verwendung eines ungesättigten Fetthydroxysäurederivats mit der allgemeinen Formel (I) nach einem der Ansprüche 1 bis 12 zur Herstellung einer dermakosmetischen Zusammensetzung, die dafür vorgesehen ist, eine Aufhellung der Haut zu bewirken.

**17.** Verwendung eines ungesättigten Fetthydroxysäurederivats entsprechend der allgemeinen Formel (I):

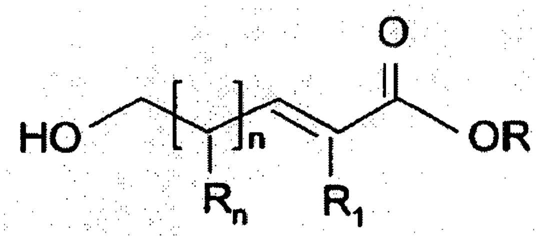

(I)

wobei Folgendes gilt:

- die $R_n$ stehen unabhängig voneinander für Folgendes: H oder eine geradkettige oder verzweigtkettige Alkylgruppe, die 1 bis 6 Kohlenstoffatome umfasst und die gegebenenfalls mit einem Halogenatom substituiert ist, insbesondere mit Fluor,
- $R_1$ steht für H, F, Cl, Br oder $CF_3$,
- R steht für H oder eine geradkettige oder verzweigtkettige Alkylgruppe, die 1 bis 6 Kohlenstoffatome umfasst und die gegebenenfalls mit einem Halogenatom substituiert ist, insbesondere mit Fluor, und
- $3 \leq n \leq 14$,

zur Herstellung einer dermakosmetischen Zusammensetzung, die gegen freie Radikale wirkend, entzündungshemmend, Juckreiz mildernd ist und/oder die zur Behandlung von Keratinisierungsstörungen und/oder zur Verbesserung der Narbenbildung vorgesehen ist.

**18.** Verwendung eines ungesättigten Fetthydroxysäurederivats entsprechend der allgemeinen Formel (I):

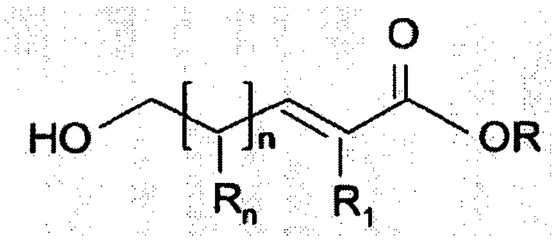

(I)

wobei Folgendes gilt:

- die $R_n$ stehen unabhängig voneinander für Folgendes: H oder eine geradkettige oder verzweigtkettige Alkylgruppe, die 1 bis 6 Kohlenstoffatome umfasst und die gegebenenfalls mit einem Halogenatom substituiert ist, insbesondere mit Fluor,
- $R_1$ steht für H, F, Cl, Br oder $CF_3$,
- R steht für H oder eine geradkettige oder verzweigtkettige Alkylgruppe, die 1 bis 6 Kohlenstoffatome umfasst und die gegebenenfalls mit einem Halogenatom substituiert ist, insbesondere mit Fluor, und
- $3 \leq n \leq 14$,

zur Herstellung einer dermakosmetischen Zusammensetzung, die zur Behandlung der Schuppenflechte, von Juckreiz und/oder der atopischen Dermatitis vorgesehen ist.

## Claims

1. Unsaturated fatty hydroxy acid derivatives corresponding to general formula (I):

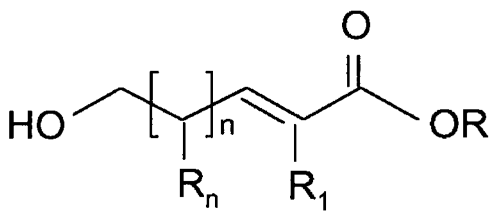

(I)

in which:

. $R_n$ independently represents one or other of H or a linear or branched alkyl group consisting of 1 to 6 carbon atoms, possibly substituted by a halogen atom, in particular fluorine.
. $R_1$ represents H, F, Cl, Br or $CF_3$,
. R represents H, and
. $3 \leq n \leq 14$,

on the condition, however, that when $n \neq 10$, at least one of the $R_1$ and $R_n$ radicals does not represent hydrogen.

2. Unsaturated fatty hydroxy acid derivatives of general formula (I) according to claim 1, wherein:

. R represents F, Cl, Br or $CF_3$.

3. Unsaturated fatty hydroxy acid derivatives of general formula (I) according to claim 1, wherein:

. $6 \leq n \leq 14$.

4. Unsaturated fatty hydroxy acid derivatives of general formula (I) according to claim 1, wherein:

. $R_1$ represents F, Cl, Br or $CF_3$,
. $R_n$ = H

5. Unsaturated fatty hydroxy acid derivatives of general formula (I) according to claim 1, wherein:

. $R_1$ represents F, Cl, Br or $CF_3$, and
. at least one of the $R_n$ radicals represents a linear or branched alkyl group consisting of 1 to 6 carbon atoms, possibly substituted by a halogen atom, in particular fluorine.

6. Unsaturated fatty hydroxy acid derivatives of general formula (I) according to claims 1 or 2, wherein:

. $R_1$ = F,
. $R_n$ = H.

7. Unsaturated fatty hydroxy acid derivatives of general formula (I) according to claim 6, wherein:

. $R_1$ = F,
. $R_n$ = H,
. n = 13 .

8. Unsaturated fatty hydroxy acid derivatives of general formula (I) according to claim 2, wherein:

. $R_1$ = F,
. $R_n$ = H,
. n = 6.

**9.** Unsaturated fatty hydroxy acid derivatives of general formula (I) according to claim 1, wherein:

. $R = R_1 = H$
. only one of the $R_n$ radicals represents a methyl group, the others being hydrogen atoms, and
. n = 4.

**10.** Unsaturated fatty hydroxy acid derivatives of general formula (I) according to claim 1, wherein:

. $R_1 = R_n = H$, and
. n = 10.

**11.** Unsaturated fatty hydroxy acid derivatives of general formula (I) according to claim 2, wherein:

. $R_1 = F$,
. $R_n = H$, and
. n = 10.

**12.** Unsaturated fatty hydroxy acid derivatives of general formula (I) according to claim 2, wherein:

. $R_1 = F$,
. $R_n = H$, and
. n = 8.

**13.** Dermocosmetologic compositions including an unsaturated fatty hydroxy acid derivative of general formula (I) according to one of claims 1 to 12, combined with a dermatologically acceptable excipient.

**14.** Use of unsaturated fatty hydroxy acid derivative of general formula (I) according to one of claims 1 to 12, for the manufacture of a dermocosmetologic composition intended for the treatment of pigmentation disorders.

**15.** Use of unsaturated fatty hydroxy acid derivative of general formula (I) according to one of claims 1 to 12, for the manufacture of a dermocosmetologic composition intended for the treatment of skin ageing and white or brown age spots.

**16.** Use of unsaturated fatty hydroxy acid derivative of general formula (I) according to one of claims 1 to 12, for the manufacture of a dermocosmetologic composition intended to produce skin whitening.

**17.** Use of unsaturated fatty hydroxy acid derivatives corresponding to general formula (I):

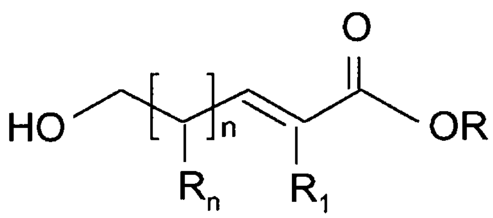

(I)

in which:

. $R_n$ independently represents one or other of H or a linear or branched alkyl group consisting of 1 to 6 carbon atoms, possibly substituted by a halogen atom, in particular fluorine.
. $R_1$ represents H, F, Cl, Br or $CF_3$,
. R represents H or a linear or branched alkyl group with 1 to 6 carbon atoms, possibly substituted by a halogen atom, particularly fluorine, and
. $3 \leq n \leq 14$,

for the manufacture of a dermocosmetologic composition with anti-radical, anti-inflammatory, anti-pruriginous activity, and/or intended for the treatment of keratinisation disorders, and/or for improving healing.

**18.** Use of unsaturated fatty hydroxy acid derivatives corresponding to general formula (I):

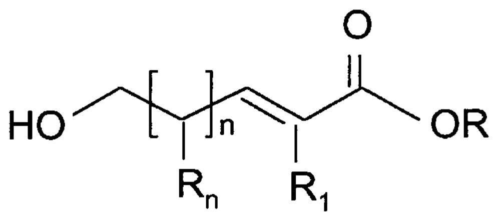

(I)

in which:

. $R_n$ independently represents one or other of H or a linear or branched alkyl group consisting of 1 to 6 carbon atoms, possibly substituted by a halogen atom, in particular fluorine.
. $R_1$ represents H, F, Cl, Br or $CF_3$,
. R represents H or a linear or branched alkyl group with 1 to 6 carbon atoms, possibly substituted by a halogen atom, particularly fluorine, and
. $3 \leq n \leq 14$,

for the manufacture of a dermocosmetologic composition intended for the treatment of psoriasis, pruritus and/or atopic dermatitis.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1679071 A
- EP 0989111 A
- EP 0568307 A


### Littérature non-brevet citée dans la description


- **EDWARD E. SMISSMAN et al.** *JOU,* 1964, vol. 29, 3517-3520
- **HANESSIAN et al.** *Can. J. Chem.,* 1987, vol. 65, 1859-1866
- **LEE et al.** *J. Org. Chem.,* 1993, vol. 58, 2918-2919
- **HURD ; SAUNDERS.** *J. Am. Chem. Soc.,* 1952, vol. 74, 5324-5328
- **KRISHNAMURTHY et al.** *Indian J. Chem. Sect. A,* 1989, vol. 28, 288-291
- **PLETTNER et al.** *J. Chem, Ecol.,* 1995, vol. 21, 1017-1030
- **SUEMATSU M et al.** Free Radicals Pratical Approach. 1996, 83-99